# EUROPEAN PATENT SPECIFICATION

(11) **EP 1 286 690 B1**
(45) Date of publication and mention of the grant of the patent: **04.07.2007**
(21) Application number: 01927645.0
(22) Date of filing: 02.05.2001
(51) Int. Cl.: A61K 38/34, A61K 38/18, A61P 9/00

(54) **USE OF ALPHA-MSH AND EPO FOR THE MANUFACTURE OF A MEDICAMENT FOR PREVENTING OR TREATING ISCHEMIC CONDITIONS**
VERWENDUNG VON ALPHA-MSH UND EPO ZUR HERSTELLUNG EINES ARZNEIMITTELS ZUR PROPHYLAXE ODER BEHANDLUNG VON DURCH ISCHÄMISCHEN ZUSTÄNDEN BEWIRKTEN KRANKHEITEN
UTILISATION D'ALPHA-MSH ET D' EPO POUR LA PREPARATION D'UN MEDICAMENT POUR PREVENIR OU TRAITER DES ETATS ISCHEMIQUES

(30) Priority: 02.05.2000 US 201264 P; 11.08.2000 DK 200001204; 22.11.2000 DK 200001757; 06.03.2001 DK 200100369
(43) Date of publication of application: 05.03.2003
(73) Proprietor: Action Pharma A/S, 8200 Arhus N (DK)
(72) Inventor: NIELSEN, Soren, DK-8230 Abyhoj (DK); FROEKIAER, Joergen, DK-8230 Abyhoj (DK); JONASSEN, Thomas, Engelbrecht, Norkild, DK-2000 Frederiksberg (DK); BJERKE, Thorbjorn, DK-3480 Fredensborg (DK)
(74) Representative: Plougmann & Vingtoft A/S
(86) International application number: PCT/DK2001/000303
(87) International publication number: WO 2001/082952

(56) References cited:
- KWON TAE-HWAN ET AL: "Reduced abundance of aquaporins in rats with bilateral ischemia-induced acute renal failure: Prevention by alpha-MSH." AMERICAN JOURNAL OF PHYSIOLOGY, vol. 277, no. 3 PART 2, 1999, pages F413-F427, XP002184347 ISSN: 0002-9513
- CHIAO H ET AL: "ALPHA-MELANOCYTE-STIMULATING HORMONE PROTECTS AGAINST RENAL INJURY AFTER ISCHEMIA IN MICE AND RATS" JOURNAL OF CLINICAL INVESTIGATION, NEW YORK, NY, US, vol. 99, no. 6, 15 March 1997 (1997-03-15), pages 1165-1172, XP000945213 ISSN: 0021-9738
- SAKANAKA M ET AL: "In vivo evidence that erythropoietin protects neurons from ischemic damage" PROCEEDINGS OF THE NATIONAL ACADEMY OF SCIENCES OF USA, NATIONAL ACADEMY OF SCIENCE. WASHINGTON, US, vol. 95, April 1998 (1998-04), pages 4635-4640, XP002142085 ISSN: 0027-8424
- STAR ROBERT A ET AL: "Evidence of autocrine modulation of macrophage nitric oxide synthase by alpha-melanocyte-stimulating hormone." PROCEEDINGS OF THE NATIONAL ACADEMY OF SCIENCES OF THE UNITED STATES, vol. 92, no. 17, 1995, pages 8016-8020, XP002184348 1995 ISSN: 0027-8424

## Description

### FIELD OF INVENTION

The present invention relates to the use of α-MSH or of an α-MSH equivalent including the sequence His-Phe-Arg and EPO for the preparation of a medicament for the prevention or treatment of an ischemic condition.

In the present specification and claims, ischemia is defined as a reduced blood flow to one or more organs resulting in a reduced oxygen delivery and/or utilization by the tissues. Acute, subacute or chronic ischemia of an organ or an extremity or a tissue can be caused by a wide variety of diseases. This includes (non-limiting list) atheromatous disease with thrombosis, embolism from the heart or from blood vessel from any organ, vasospasm, aortic aneurysm or aneurisms in other organs, thoracal or abdominal or dissecting aortic aneurysm, hypotension due to heart disease, hypotension due to systemic disease including infection or allergic reactions, hypotension due to one or more toxic compounds or poison(s) or drug(s). Moreover the non-limiting list include ischemia secondary to one or more of the following diseases and condtions: diabetes mellitus, hyperlipidaemia, thromboangiitis obliterans (Buerger's disease), Takayasu's syndrome, arteritis temporalis, mucocutaneous lymph node syndrome (Kawasaki disease), cardiovascular syphilis, connective tissue disorders as Raynaud's disease, phlegmasia coerulae dolens, blood vessel trauma including iatrogene trauma such as cannulation or surgery or organtransplantation. Moreover the list includes ischemia caused by surgery of one or more organs, transplantation of one or more organs, surgical insertion transplants, devices, grafts, prostheses or other biomedical compounds or devices. Finally the list also includes ischemia due to septic chock or conditions associated with systemic hypotension. Ishemia may occur in one or more organs including (non-listing list): brain, heart, extremities, kidney, spleen, liver, intestine, stomach, lung, eye, skin, muscles, pancreas, endocrine organs and others.

Ischemia induces by reduced/complete arrest in arterial blood supply multiple tissue reactions including neutrofil accumulation, other inflammatory responses and cell death. Ischemia is involved in multiple diseases, associated with major surgery and secondary to other severe diseases. Identification of compounds that could inhibit or prevent (either completely or partially) many of the cell/tissue/organ impairments or destructions occurring as a result of ischemia would be of great benefit. In the present application is described a series of studies, which have established α-MSH and epoetin with such properties in distinct models (ischemia induced renal failure, myocardial ischemia and intestinal ischemia).

### BACKGROUND OF THE INVENTION

Acute myocardial infarction (AMI) is one of the most common causes of death in the developed countries. The incidence of AMI in a country as Denmark with 5,000,000 citizens, is 19,000. The pre-hospital mortality is 15%, which means that approximately 16,000 patients are hospitalized with AMI every year. The acute mortality among these patients is 20%. The one-year mortality among patients who leave hospital is 5-10%.

AMI almost always occurs in patients with coronary atheroma because of sudden coronary thrombosis. Today, fibrinolytic therapy and primary percutaneous transluminal coronary angioplasty (PTCA) are standard treatments and can achieve early reperfusion in 50-70% of patients (spontaneous reperfusion rate is less than 30%). However, early intervention is necessary in order to reduce the ischemic damages and patients with AMI still develop irreversible ischemic myocardial damages with the development of impaired myocardial function. The long term-prognosis after AMI has shown to be directly correlated to left-ventricular function evaluated through eccocardiographic measurement of left ventricular ejection fraction. Identification of compounds that could inhibit or prevent (either completely or partially) many of the cell/tissue/organ impairments or destructions occurring as a result of ischemia in the infarcted and reperfused myocardium would be attractive tools in inter alia reducing the irreversible damages that eventually result in impaired myocardial function after AMI.

Ischemia of the kidney is frequently seen as a consequence of reduced blood pressure, hypovolemia, blood loss during surgery or associated with septicemia. This results in ischemia-induce acute renal failure which for a large fraction deteriorate into chronic renal failure. Currently no efficient treatment exists to prevent the development of renal failure.

Acute ischemia of the small intestine is a condition with a high mortality. The most common reasons are an embolia or an atherosclerotic thrombosis in the superior mesenteric artery. The initial symptoms are often discrete which means that the condition is difficult to diagnose, and many patients develop severe hemodynamic disturbances with shock symptoms. The treatment is resection of the ischemic intestine or revascularisation combined with pressure support. However, the prognosis is poor with a lethality higher than 75%. Since surgical resection in most cases involves the whole ileum and the ascending part of the colon, the surviving patients will often suffer from severe malabsorption. An alternative procedure to intestinal resection is surgical revascularisation, however the intestine is often severely damaged at the time of surgery and therefore a second intervention with resection of non-vital intestine is needed. Treatments that significantly could increase the prognosis during conditions with acute intestinal ischemia are therefore wanted.

### BRIEF DISCLOSURE OF THE INVENTION

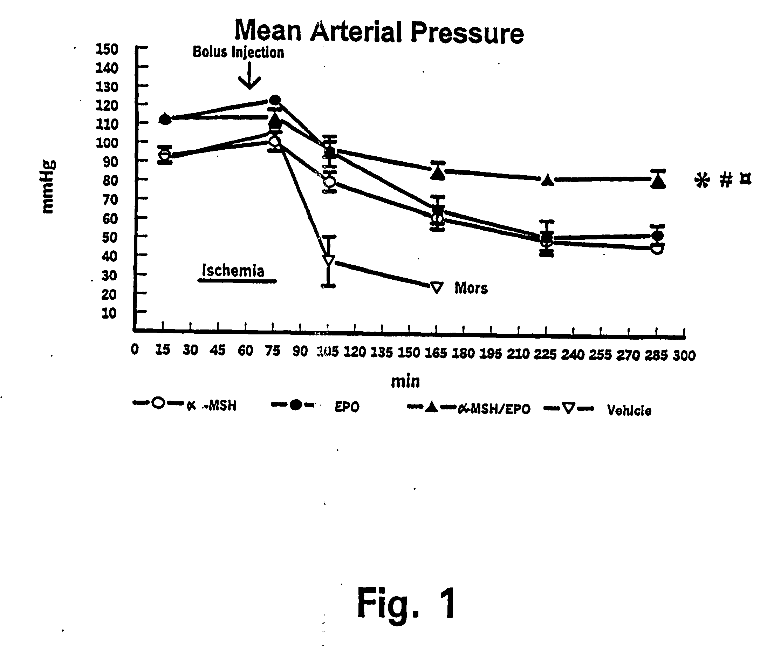

The potential beneficial role of α-MSH and EPO combined treatment with both compounds in AMI has never been established. Therefore, the present inventors have performed a series of experiments in a model of coronary ischemia/reperfusion in rats. The role of i.v. treatment with α-MSH (200 µg/day/kg body weight), EPO in two different doses (200 and 1000 U/day/kg body weight) and, finally, the role of combined treatment with α-MSH (200 µg/day/kg body weight) and low dose rh-EPO (200 U/day/kg body weight) on myocardial remodeling and cardiac performance after sixty minutes ischemia were investigated. It was found that the high dose Rh-EPO treatment, α-MSH treatment and combined treatment with low dose rh-EPO and α-MSH all prevented death in the ischemic and reperfused animals. Mortality was 45 % in vehicle treated rats. Furthermore the treatments significantly decreased the size of the infarction that developed in the ischemic and reperfused myocardium. Briefly the findings were as follows: The mean area of risk was similar in all ischemia/reperfusion groups when the hearts were examined two days after reperfusion (figure 1). Vehicle treated rats had an infarct size of 67±7% of the area of risk. Low dose rh-EPO did not significantly reduce the size of the infarcted part of the area of risk. However, high dose rh-EPO as well as α-MSH significantly reduced infarction size. High dose Rh-EPO reduced the infarction size by 65% and α-MSH reduced the infarction size by 58% compared to the vehicle treated animals. Combined treatment with low dose rh-EPO and α-MSH reduced the size of the infarction size by 75%. The sham-operated time-control animals had no infarction (data not shown).

Clamping of the coeliac trunk and the superior mesenteric artery in rats induce severe splanchnic ischemia. An experimental model in which a 45 minut period of splanchic ischemia is followed by reperfusion results in irreversible circulatory failure and shock (splanchnic artery occlusion shock (SAO-shock)). The average survival rate after reperfusion is less than three hours. At the present no known treatment is able to reverse the SAO-shock. It has recently been shown that acute i.v. treatment with high-dose recombinant human erythropoietin (rh-EPO)(1.000 U/ kg body weight) or the melanocortin peptide ACTH-(1-24) (160 µg/kg body weight) to some degree were able to postpone death in a rat model of SAO-Shock. Both treatments were unable to restore the pre-SAO blood pressure and the studies gave no information about perfusion of vital organs as the kidneys. In fact the acute mortality (death within 4 hours) was still high (20-30 %) in both rh-EPO and ACTH-(1-24) treated animals (F. Squadrito et al. BJP 127: 482-88, 1999 and F. Squadrito et al. BJP 128: 816-822, 1999).

The present inventors have investigated the difference in the way that the two compounds act in SAO-shock, and have found out that combined treatment with re-EPO and a melanocortin could have major advances in order to keep mean arterial blood pressure (MAP) and preserve vital organ function as renal perfusion and glomerular filtration rate (GFR). The inventors have therefore examined the effect of combined treatment with low dose rh-EPO (200 U/kg body weight)(i.e five times less than the dose used by Squadrito et al.) and α-melanocyte stimulation hormone (α-MSH) (200 µg/kg body weight) on MAP, GFR and acidosis. It was found that combined rh-EPO and α-MSH treatment completely prevented death during the study period (Mortality in the vehicle treated rats was 100%), and protected against severe hypotension, acute renal failure and severe metabolic acidosis. These beneficial effects were not present to the same extent in rats treated with either rh-EPO or α-MSH alone. In fact the animals treated with either rh-EPO or α-MSH alone had severe hypotension and acidosis with marked decreased renal function. GFR was reduced by 71% in rats treated with rh-EPO and by 91% in rats treated with α-MSH, but only with 32% in the rats that were treated with the comibantion of rh-EPO and α-MSH.

Urinary concentration and dilution depends on the presence of a discrete segmental distribution of transport properties along the renal tubule. Concentration of the urine requires 1) establishment and maintenance of a hypertonic medullary interstitium and 2) vasopressin regulated water transport across the collecting duct epithelium for osmotic equilibration. Thus, defects in any of these mechanisms would be predicted to be associated with urinary concentrating defects. The aquaporins (AQPs) are a family of membrane proteins that function as water channels. AQP1 is highly abundant in the proximal tubule and descending thin limb and several studies have emphasized its important role in the constitutive water reabsorption in these segments and its role for urinary concentration. In the kidney collecting duct at least three aquaporins are known to be expressed and they participate in the vasopressin-regulated water reabsorption. AQP2 is the apical water channel of collecting duct principal cells and is the chief target for regulation of collecting duct water permeability by vasopressin. Water transport across the basolateral plasma membrane of collecting duct principal cells is thought to be mediated by aquaporin-3 (AQP3) and aquaporin 4 (AQP4). A series of studies have demonstrated that altered expression and apical targeting of AQP2 play a significant role in water balance disorders. Along the nephron a number of renal sodium transporters are expressed including NH3, NaPi-2, and Na,K-ATPase in the proximal tubule and BSC-1, TSC and Na,K-ATPase in the distal tubule. They are all essential for urinary concentration and renal function.

Ischemia-induced experimental acute renal failure (ARF) induced by ischemia and reperfusion in rats is known to cause characteristic structural alterations in renal tubule epithelia in association with an impairment of urinary concentrating mechanism. The straight portion of proximal tubule (S3 segment) and thick ascending limb (TAL), which both are located in the outer medulla of the kidney, are marginally oxygenated under normal conditions and suffers the most severe and persistent hypoxia after an ischemic injury. Therefore, the proximal tubule (S3 segment) and TAL are generally viewed as the main sites of ischemic insult in the kidney. In contrast, collecting ducts are generally considered to be relatively invulnerable to ischemic injury. This ARF model provides an appropriate setting to evaluate the effect of α-MSH (α-melanocyte stimulating hormone) in ischemia-induced injury, allowing test in quantitative terms and in a direct way the effect of α-MSH treatment on renal expression of aquaporins, sodium transporters and renal functional parameters in a quantitative fashion. Initial studies of α-MSH treatment in ARF indicated some effect but lack important quantitative aspects and did not address the effect on kidney function and the expression of renal transporters (Chiao, H et al. J.Clin.Invest. 99:1165-1172,1997.)

### DETAILED DISCLOSURE OF THE INVENTION

### Melanocortins

Melanocortins are proopiomelanocortin-derived mammalian peptide hormones that include adrenocorticotropic hormone [ACTH (1-39)], α- melanocyte-stimulating hormone [α-MSH (1-13)], and related amino acid sequences including β and γ-MSH. Melanocortin peptides have potent antiinflammatory/anticytokine activity (Lipton and Catania Immunol.Today, 18: 140-145, 1997). Melanocortins excert at least some of their effect via stimulation of melanocortin receptors. For melanocyte stimulating hormones (MSH) the action is in part ascribed to binding and activation of type 1-5 melanocortin receptors (MC1-MC5).

Melanocortins have a variety of functions including immunomodulation, anti-inflammation, body temperature regulation, pain perception, aldosterone synthesis, blood pressure regulation, heart rate, vascular tone, brain blood flow, nerve growth, placental development, synthesis/release of a variety of hormones such as aldosterone, thyroxin, prolactin, FSH. ACTH has a major effect on stimulating steroidoneogenesis. Also α-MSH induces pigment formation in skin.

Five genes encoding melanocortin receptor subtypes have been identified (MC-receptor type 1-5). The MC receptors belong to the class of G-protein coupled receptors and have seven membrane spanning domains. All receptor subtypes involve increased production of camp to excert their actions. Type 2 receptor (MC2) represents the ACTH receptor whereas the other subtypes are melanocyte stimulating hormone receptors (MSH-receptors).

A series of studies have been performed on the MC receptors in a variety of tissues. Type 1 receptor (MC1), to which α-MSH binds with great affinity, is known to be expressed in several tissues and cells such a brain, including astrocytes, testis, ovary, macrophages, neutrophils. However MC1 is likely to be expressed in an even wider range of tissues although this remains to be established. The selectivity for the MC receptors to bind different melanocortin peptides vary. MC1 binds with great affinity α-MSH and with lower affinity also β-MSH, γ-MSH and ACTH. MC2 has been reported only to bind ACTH but non of the MSH peptides. The highest affinity for the ligands of the other receptors including γ-MSH (MC3-receptor), β-MSH (MC4-receptor). In contrast MC5 binds with much lower affinity the MSH peptides but with the same pattern as MC1 (i.e. highest affinity for α-MSH).

It is important to emphasize that a number of actions of MSH peptides, especially α-MSH, are not fully established with respect to which receptors are involved. The anti-inflammatory action of α-MSH has been speculated to involve a variety of processes including interference with NO production, endothelin-1 action, interleucin 10 formation, which again is linked to MC1 receptors expressed in macrophages, monocytes.

α-MSH has also been shown to be important in a variety of inflammatory processes (Lipton and Catania 1997): 1) Inhibit chemotactive migration of neutrophils (Catania1996). 2) α-MSH including analogues inhibit the release of cytokine (IL-1, TNF-α) in response to LPS treatment (Goninard1996). 3) Inhibit TNF-α in response to bacterial endotoxin (Wong, K.Y. et al. Neuroimmunomodulation, 4: 37-41, 1997). 4) ICV or IP administration of α-MSH inhibit central TNF-α production by locally administered LPS. 5) α-MSH has been shown to reduce the inflammation in experimental inflammatory bowel disease (Rajora, N. et al. Peptides, 18: 381-385, 1997), ishemia-induced acute renal failure(Star, R A. et al. Proc.Natl.Acad.Sci.U.S.A, 92: 8016-8020, 1995). 6) α-MSH also has some protective effect by inhibiting the induction and elicitation of contact hypersensitivity and induces hapten tolerance, and it is speculated that α-MSH may mediate important negative regulation of cutaneous inflammation and hyperproliferative skin diseases (Luger, T.A. J.Investig.Dennatol.Symp.Proc., 2: 87-93, 1997. To this end α-MSH causes increased IL-8 release from dermal microvasculature endothelial cells (Hartmeyer, M. J.Immunol., 159: 1930-1937, 1997).

### Erythropoetin (EPO)

The cellular adaptation to hypoxia involves many changes in gene expression, such as those of erythropoietin (Epo), vascular endothelial growth factor (VEGF), glycolytic enzymes, and tyrosine hydroxylase. Several reports have demonstrated that both oxygen sensing and chemical signaling occur via a common pathway that leads to the activation of hypoxia-inducible factor-1 (HIF-1), a transcription factor which is induced over a physiologically relevant range of oxygen tensions Epo is a 34-kDa glycoprotein hormone which has been characterized as the principal regulator of erythropoiesis and was thought to be exclusively produced in fetal liver and adult kidney in response to hypoxia . The molecular biology of the oxygen sensing mechanism underlying the transcriptional activity of Epo has been intensively investigated in HepG2 and Hep3B human hepatoma cell lines. In addition to transcriptional activation by HIF-1, mRNA stabilization has been found to account for an accumulation of Epo mRNA. Agents such as cobalt chloride (CoCl2) and desferrioxamine (DFX) are able to mimic the hypoxia-induced Epo transcription.

Indirect evidence has been provided to indicate that redox-mediated processes are likely to be involved in the induction of the EPO gene. Thus, iron and reactive oxygen species might play a critical role in the oxygen sensing mechanisms involved in the regulation of the expression of the EPO gene. Recent reports suggest that, along with its role in erythropoiesis. EPO might be of biological significance in the central nervous system. In vivo, EPO mRNA is expressed in both rodent and primate brain tissues and its expression is increased following hypoxia. Taken together, several findings imply that EPO acts on neurons in a paracrine way. This notion nas been supported by the *in vitro* and *in vivo* neuroprotective effects of Epo. Several groups (Sadamoto, Y. et al. Biochem. Biophys. Res. Commun., 253: 26-32, 1998, Sakanaka, M. et al. Proc.Natl.Acad.Sci.U.S.A, 95: 4635-4640, 1998, Bernaudin, M. et al. J.Cereb.Blood Flow Metab, 19: 643-651, 1999) have shown that the direct administration of EPO to the central nervous system of mice, rats, and gerbils to some extent reduces neuronal death and prevents learning disability associated with cerebral ischemia.

In its broadest embodiment, the present invention relates to the use of α-MSH or of an α-MSH equivalent including the sequence His-Phe-Arg and EPO the preparation of a medicament for the treatment or prevention of an ischemic condition in an organ of a mammal. In the present specification and claims, the term "treatment according to the invention" will generally include treatment of an existing condition as well as prevention of such condition (prophylactic treatment) unless the text specifically excludes this interpretation.

In its broadest concept the invention relates to any condition wherein the normal function of the organs or tissues is altered due to ischemia. The injury may include acute and chronic injury. Chronic injury includes situations of repetitive injuries alternating with periods of complete or partial recovery of the organ(s) or tissue(s) function. Individual represents humans as well as other mammals.

According to the present invention it has surprisingly been found that treatment with α-MSH and low dose rh-EPO in combination had an extraordinary surprising synergistic effect dramatically preventing death or organ dysfunction/deterioation in a number of conditions associated with organ ischemia. More specifically the treatment 1) dramatically prevented death and reduced infarction size of ischemic myocardium in heart diseases, 2) significantly prevented severe deterioation of kidney function in ischemic kidney and urinary tract diseases, and 3) dramatically prevented death and failure of various organs in ischemic intestinal disease. Moreover it was found that treatment with either α-MSH or rh-EPO had a significant effect on the above conditions which thus provide surprising novel methods of using α-MSH, α-MSH equivalents and EPO separately for prevention or treatment of some of the conditions mentioned herein.

In one important aspect of the invention, the condition to be treated is due to or caused by ischemia of the tissue such as in arterial stenosis or any other complete or partial restriction in blood supply. The ischemia may be acute or chronic depending on the severity of the disease and, furthermore, the condition may be reversible or irreversible. An example of a reversible condition may be due to fall in the blood pressure during surgery or other intervention, which affect the blood perfusion of the organ. Accordingly, the condition to be treated may be any decrease in systemic blood flow such as hypotension, which may affect the systemic blood flow to the intestine, heart kidney or any other organ.

The use according to the invention may be of special benefit in relation to conditions caused by or associated with transplantation of any organ or vessel, including prevention of graft versus host reaction. In such conditions, the entire organ is extremely sensitive to all alterations with respect to nutrition, metabolism, perfusion etc., and the treatment according to the present invention is believed to stabilize the condition and make the tissue more resistant to any situation stressing the function of the organ. The use according to the present invention also encompasses the use of an effective dose of α-MSH and/or of an α-MSH equivalent including the sequence His-Phe-Arg and EPO to the organ transplant during transport to the recipient, including addition of an effective dose of α-MSH and/or of an α-MSH equivalent including the sequence His-Phe-Arg and EPO to the transportation medium.

The invention also relates to the combination treatment with α-MSH and/or an α-MSH equivalent and including the sequence His-Phe-Arg and EPO in view of the evidence that the effects are surprisingly dramatic e.g. in myocardial infarction.

One of the most common heart conditions is intermittent angina or chest pain wherein the treatment according to the invention may be of special interest. Conditions relating to angina include unstable angina, stable angina, Prinzmetal's variant angina, and Syndrome X.

In a further aspect, the prevention and treatment may be utilized in situations caused by pericarditis, myocardiel infarction, myocardial ischemia, myocarditis, myxodemia, and endocarditis.

The condition to be treated can be associated with cardial arrhythmia. Either as the primary disease or secondary to another condition of the individual. Examples of miscellaneous causes of arrhythmia include acute infections particularly those affecting the lungs, pulmonary embolism, hypotension, shock, anoxaemia or anaemia which can precipitate myocardial ischemia and thus cause arrhythmia. The arrhythmia will aggravate the circulatory disturbance and thereby set up a vicious, self-perpetuating cycle.

It is believed that the use according to the present invention will increase the threshold for development of arrhythmia thus preventing the development of the arrhythmia. The effect may by directly on the conduction system or indirectly by acting on a condition tricking or being the cause of the arrhythmia.

In a syndrome or an arrhythmia which can be alleviated according to the present invention may be either primary or secondary and may be selected from ventricular or supra ventricular tachyarrhythmias, atrioventricular block, sinus node disease, Wolff-Parkinson-White syndrome, Lenégres disease, Lev's disease any syndrome involving an abnormal myocardial connection between atrium and ventricle.

Antiarrhythmic therapy performed with the aim of suppressing an arrhythmia is always associated with a risk of creating new arrhythmias. The arrhythmias may occur as a toxie reaction due to an overdose of the drug. However, particularly during treatment with the group of drugs known as Class IA drugs, arrhythmias can occur as a non dosage-dependent side effect - an idiosyncratic reaction - developing at drug concentrations well within the therapeutic range. According to a further embodiment, the condition may be caused by one or more antiarrhytmic drugs including, digitalis, quinidine, disopyramide, adenosin, aprindine, flecainide, amiodarone, sotalol, meciletine, beta blocking agents, and verapamil.

It is contemplated that treatment with the combination according to the present invention will decrease the risk of developing arrythmias due to concommittant treatment with other antiarrythmic medicament(s).

In a further aspect of the invention, the condition may be characterised by one or more abnormalities as measured by electrocardiography (ECG). The abnormality on the ECG may relate to an alteration selected from one or more changes in the configuration selected from the P wave, the ST segment, the T wave, the QRS complex, the Q wave, the delta wave, and the U wave.

Other conditions which may be alleviated by administration of an effective dose of α-MSH and/or of an α-MSH equivalent including the sequence and EPO are the effect of electrolyte derangement on the organ (e.g. the heart) as well as the derangement itself, including abnormalities in the relative concentrations of individual ions one to another. Such condition includes an abnormal serum concentration of one or more of the electrolytes selected from the group consisting of potassium, calcium, sodium, and magnesium

According to the present invention, the tissue that may be affected includes one or more cell types present in the organ and may be selected from epithelial celts, macrophages, the reticulo endothelial system monocytes, neutrophil granulocytes, eosinophil granulocytes, basophil granulocytes, T-cells, B-cells, mast cells, and dendritic cells. Especially, the T-cells, B-cells, and mast cells may be of certain interest in this respect.

A preferred aspect of the invention relates to prevention or treatment wherein a dose of α-MSH or of an α-MSH equivalent including the sequence His-Phe-Arg and EPO is to be administered prophylactically for preventing a progress of the condition or of any symptom of the condition.

A preventive or prophylactic treatment may be an ongoing treatment during e.g. surgery or for the prevention of heart attacks in a patient suffering from coronary stenosis. The preventive treatment may also be for a limited period. The skilled person will be able to evaluate the specific treatment schedule based on the actual situation. In a preferred embodiment, the treatment or prevention according to the present invention is able to reduce the infarction size upon ischemia of the coronary arteries. Such infarction size may be reduced by 20%, such as at least 30%, preferably by at least 50% compared to the untreated individual as will appear from the example herein.

Accordingly, the dose of α-MSH or of an α-MSH equivalent including the sequence His-Arg-Phe and EPO is to be administered prophylactically for prevention of the establishment of the condition or of any symptom of the condition.

The dose of α-MSH or of an α-MSH equivalent including the sequence His-Phe-Arg and EPO according to the invention may be administered as a single dosage, regular or continued administration, or as a sequential administration.

The administration may be parenteral administration, such as intraperitoneal administration, intrathecal administration, systemic administration, local administration including use of drug target systems and implants, topical administration, transmucosal administration, transdermal administration and/or oral administration.

Accordingly, the administration can include systemic administration; injection into tissue or into a body cavity including joints; implantation into tissue or into a body cavity; topical application to the skin or to any gastrointestinal surface, or to a mucosal surface including the lining of body cavities.

In a further important aspect, the α-MSH equivalent is a polypeptide having at least 3 amino acids including the following sequence His-Phe-Arg, and being able to act on an α-MSH receptor.

In a preferred embodiment, the α-MSH, α-MSH equivalent including the sequence His-Phe-Arg, EPO are in the form of the recombinantly produced proteins.

A very important aspect of the present invention is the beneficial effect of the combination of an α-MSH and/or an α-MSH equivalent including the sequence His-Phe-Arg with EPO as the combination has an additive effect on the condition to be treated. An additive effect may be measured as an effect increasing any of the effects of the individual drugs used in the same dosage. Additionally, it is believed that the combination in most circumstances will have a synergistic effect. A synergistic effect may be measured as an effect increasing the sum of the individual effect of each of the individual drugs used in the same dosage. A synergistic effect may also include the situation where an effect equal to the sum of effects by the individual treatments is obtained with minor doses of the individual drugs than when used in combination.

With respect to the combination it is possible to obtain a synergistic effect where the EPO and the α-MSH or α-MSH equivalent including the sequence His-Phe-Arg is to be administered independently of each other. The time span from the release of one of the active ingredients to the organ or tissue in question until the other active ingredient is subjected to the tissue or organ may be several days, such as even 5 days, or one week. However, the drugs are preferably to be administered within at least 48 hours, preferably within 24 hours, such as within 12 hours. However, the practical reasons the active ingredients are normally to be substantially co-administered.

### Pharmaceutical formulations and compositions:

In the following examples of suitable compositions containing α-MSH or an α-MSH equivalent including the sequence His-Phe-Arg and recombinantly produced EPO are given. Depending on the use of the α-MSH and/or an α-MSH equivalent including the sequence His-Phe-Arg and EPO, a composition is a pharmaceutical composition.

For the administration to an individual (an animal or a human) the substance(s) are preferably formulated into a pharmaceutical composition containing the substance(s) and, optionally, one or more pharmaceutically acceptable excipients.

The compositions may be in form of, e.g., solid, semi-solid or fluid compositions such as, *e.g.,* but not limited to bioabsorbable patches, drenches, dressings, hydrogel dressings, hydrocolloid dressings, films, foams, sheets, bandages, plasters, delivery devices, implants, powders, granules, granulates, capsules, agarose or chitosan beads, tablets, pills, pellets, microcapsules, microspheres, nanoparticles, sprays, aerosols, inhalation devices,gels, hydrogels, pastes, ointments, creams, soaps, suppositories, vagitories, tooth pastes, solutions, dispersions, suspensions, emulsions, mixtures, lotions, mouthwashes, shampoos, enemas,

kits containing e.g. two separate containers, wherein the first one of the containers contains the α-MSH and/or α-MSH equivalent including the sequence and recombinantly produced EPO and/or pharmaceutically acceptable excipients and the second container containing a suitable medium intended to be added to the first container before use in order to obtain a ready-to-use composition; and in other suitable forms such as, e.g., implants or coating of implants or in a form suitable for use in connection with implantation or transplantation.

The compositions may be formulated according to conventional pharmaceutical practice, see, e.g., "Remington: The science and practice of pharmacy" 20th ed. Mack Publishing, Easton PA, 2000 ISBN 0-912734-04-3 and "Encyclopedia of Pharmaceutical Technology", edited by Swarbrick, J. & J. C. Boylan, Marcel Dekker, Inc., New York, 1988 ISBN 0-8247-2800-9.

A pharmaceutical composition comprising an active substance serves as a drug delivery system. In the present context the term "drug delivery system" denotes a pharmaceutical composition (a pharmaceutical formulation or a dosage form) which upon administration presents the active substance to the body of a human or an animal. Thus, the term "drug delivery system" embraces plain pharmaceutical compositions such as, e.g., creams, ointments, liquids, powders, tablets, etc. as well as more sophisticated formulations such as sprays, plasters, bandages, dressings, devices, etc.

Apart from α-MSH and/or an α-MSH equivalent including the sequence His-Phe-Arg and recombinantly EPO a pharmaceutical composition for use according to the invention may comprise pharmaceutically acceptable excipients.

The choice of pharmaceutically acceptable excipients in a composition for use according to the invention and the optimum concentration thereof cannot generally be predicted and must be determined on the basis of an experimental determination thereof. Also whether a pharmaceutically acceptable excipient is suitable for use in a pharmaceutical composition is generally dependent on which kind of dosage form is chosen. However, a person skilled in the art of pharmaceutical formulation can find guidance in e.g., "Remington: The science and practice of pharmacy" 20th ed. Mack Publishing, Easton PA, 2000 ISBN 0-912734-04-3.

A pharmaceutically acceptable excipient is a substance, which is substantially harmless to the individual to which the composition will be administered. Such an excipient normally fulfils the requirements given by the national drug agencies. Official pharmacopeias such as the British Pharmacopeia, the United States of America Pharmacopeia and the European Pharmacopeia set standards for well-known pharmaceutically acceptable excipients

In the following is given a review on relevant pharmaceutical compositions for use according to the invention. The review is based on the particular route of administration. However, it is appreciated that in those cases where a pharmaceutically acceptable excipient may be employed in different dosage forms or compositions, the application of a particular pharmaceutically acceptable excipient is not limited to a particular dosage form or of a particular function of the excipient.

### Parenteral compositions:

For systemic application, the compositions according to the invention may contain conventionally non-toxic pharmaceutically acceptable carriers and excipients including microspheres and liposomes.

The compositions for use according to the invention include all kinds of solid, semisolid and fluid compositions. Compositions of particular relevance are e.g. solutions, suspensions, emulsions, gels, implantation tablets and implants.

The pharmaceutically acceptable excipients may include solvents, buffering agents, preservatives, humectants, chelating agents, antioxidants, stabilizers, emulsifying agents, suspending agents, gel-forming agents, diluents, disintegrating agents, binding agents, lubricants and wetting agents. For examples of the different agents see below.

### Topical, trans-mucosal and trans-dermal compositions:

For application to the mucosa or the skin, the compositions for use according to the invention may contain conventionally non-toxic pharmaceutically acceptable carriers and excipients including microspheres and liposomes.

The compositions for use according to the invention include all kinds of solid, semi-solid and fluid compositions. Compositions of particular relevance are e.g. pastes, ointments, hydrophilic ointments, creams, gels, hydrogels, solutions, emulsions, suspensions, lotions, liniments, resoriblets, suppositories, enema, pessaries, moulded pessaries, vaginal capsules, vaginal tablets, shampoos, jellies, soaps, sticks, sprays, powders, films, foams, pads, sponges (e.g. collagen sponges), pads, dressings (such as, e.g., absorbent wound dressings), drenches, bandages, plasters and transdermal delivery systems.

The pharmaceutically acceptable excipients may include solvents, buffering agents, preservatives, humectants, chelating agents, antioxidants, stabilizers, emulsifying agents, suspending agents, gel-forming agents, ointment bases, suppository bases, penetration enhancers, perfumes, skin protective agents, diluents, disintegrating agents, binding agents, lubricants and wetting agents. For examples of the different agents see below.

### Oral compositions:

For application to the mucosa or the skin, the compositions for use according to the invention may contain conventionally non-toxic pharmaceutically acceptable carriers and excipients including microspheres and liposomes.

The composition for use according to the invention includes all kinds of solid, semi-solid and fluid compositions. Compositions of particular relevance are e.g. solutions, suspensions, emulsions, uncoated tablets, modified-release tablets, gastro-resistant tablets, orodispersible tablets, effervescent tablets, chewable tablets, soft capsules, hard capsules, modified release capsules, gastro-resistant capsules, uncoated granules, effervescent granules, granules for the preparation of liquids for oral use, coated granules, gastro-resistant granules, modified-release granules, powders for oral adminstration and powders for the preparation of liquids for oral use.

The pharmaceutically acceptable excipients may include solvents, buffering agents, preservatives, humectants, chelating agents, antioxidants, stabilizers, emulsifying agents, suspending agents, gel-forming agents, diluents, disintegrating agents, binding agents, lubricants, coating agents and wetting agents. For examples of the different agents see below.

### Examples of various agents:

Examples of solvents are but not limited to water, alcohols, vegetable or marine oils (e.g. edible oils like almond oil, castor oil, cacao butter, coconut oil, com oil, cottonseed oil, linseed oil, olive oil, palm oil, peanut oil, poppyseed oil, rapeseed oil, sesame oil, soybean oil, sunflower oil, and teaseed oil), mineral oils, fatty oils, liquid paraffin, polyethylene glycols, propylene glycols, glycerol, liquid polyalkylsiloxanes, and mixtures thereof.

Examples of buffering agents are but not limited to citric acid, acetic acid, tartaric acid, lactic acid, hydrogenphosphoric acid, diethylamine etc.

Examples of preservatives for use in compositions are but not limited to parabens, such as methyl, ethyl, propyl p-hydroxybenzoate, butylparaben, isobutylparaben, isopropylparaben, potassium sorbate, sorbic acid, benzoic acid, methyl benzoate, phenoxyethanol, bronopol, bronidox, MDM hydantoin, iodopropynyl butylcarbamate, EDTA, benzalconium chloride, and benzylalcohol, or mixtures of preservatives.

Examples of humectants are but not limited to glycerin, propylene glycol, sorbitol, lactic acid, urea, and mixtures thereof.

Examples of chelating agents are but not limited to sodium EDTA and citric acid.

Examples of antioxidants are but not limited to butylated hydroxy anisole (BHA), ascorbic acid and derivatives thereof, tocopherol and derivatives thereof, cysteine, and mixtures thereof.

Examples of emulsifying agents are but not limited to naturally occurring gums, e.g. gum acacia or gum tragacanth; naturally occurring phosphatides, e.g. soybean lecithin; sorbitan monooleate derivatives; wool fats; wool alcohols; sorbitan esters; monoglycerides; fatty alcohols;, fatty acid esters (e.g. triglycerides of fatty acids); and mixtures thereof.

Examples of suspending agents are but not limited to celluloses and cellulose derivatives such as, e.g., carboxymethyl cellulose, hydroxyethylcellulose, hydroxypropylcellulose, hydroxypropylmethylcellulose, carraghenan, acacia gum, arabic gum, tragacanth, and mixtures thereof.

Examples of gel bases and viscosity-increasing are but not limited to liquid paraffin, polyethylene, fatty oils, colloidal silica or aluminium, zinc soaps, glycerol, propylene glycol, tragacanth, carboxyvinyl polymers, magnesium-aluminium silicates, Carbopol®, hydrophilic polymers such as, e.g. starch or cellulose derivatives such as, e.g., carboxymethylcellulose, hydroxyethylcellulose and other cellulose derivatives, water-swellable hydrocolloids, carragenans, hyaluronates (e.g. hyaluronate gel optionally containing sodium chloride), and alginates including propylene glycol aginate.

Examples of ointment bases are but not limited to beeswax, paraffin, cetanol, cetyl palmitate, vegetable oils, sorbitan esters of fatty acids (Span), polyethylene glycols, and condensation products between sorbitan esters of fatty acids and ethylene oxide, e.g. polyoxyethylene sorbitan monooleate (Tween).

Examples of hydrophobic ointment bases are but not limited to paraffins, vegetable oils, animal fats, synthetic glycerides, waxes, lanolin, and liquid polyalkylsiloxanes.

Examples of hydrophilic ointment bases are but not limited to solid macrogols (polyethylene glycols).

Examples of powder components are but not limited to alginate, collagen, lactose, powder which is able to form a gel when applied to a wound (absorbs liquid/wound exudate).

Examples of diluents and disintegrating agents are but not limited to lactose, saccharose, emdex, calcium phosphates, calcium carbonate, calcium sulphate, mannitol, starches and microcrystaline cellulose.

Examples of binding agents are but not limited to saccharose, sorbitol, gum acacia, sodium alginate, gelatine, starches, cellulose, sodium coboxymethylcellulose, methylcellulose, hydroxypropylcellulose, polyvinylpyrrolidone and polyetyleneglycol.

Examples of wetting agents are but not limited to sodium laurylsulphate and polysorbate 80.

Examples of lubricants are but not limited to talcum, magnesium stearate, calcium stearate, silicium oxide, precirol and polyethylenglycol.

Examples of coating agents are but not limited to hydroxypropylcellulose, hydroxypropylmethylcellulose, polyvinylpropylidone, ethylcellulose and polymethylacrylates.

Examples of suppository bases are but not limited to oleum cacao, adeps solidus and polyethylenglycols.

The α-MSH and/or α-MSH equivalent including the sequence His-Phe-Arg and EPO may be present in the medicament in an amount of 0.001-99%, typically 0.01-75%, more typically 0.1-20%, especially 1-15% such as 1-10% by weight of the medicament.

The dose depends on the conditions to be treated. The individual drugs may be used in the doses known in the art. However, according to the present invention minor doses will generally be sufficient. With respect to the use of the combination, the EPO may very often be effective in rather small doses. The necessary dose of EPO in the combination may be such a dose which, when used alone, would not have any significant effect on the condition.

It is contemplated that the dose of α-MSH and/or α-MSH equivalent including the sequence His-Phe-Arg will be in the range of 1 ng to 100 mg pr. kg body weight, typically 1µg to 10 mg pr. kg body weight, more typically 10µg to 1 mg pr. kg body weight, such as 50-500 µg pr. kg body weight; and that the dose of EPO will be in the range of 0,001-10000 IU pr. kg body weight, typically 0,1-5000 IU pr. kg body weight, more typically 1-1000 IU pr. kg body weight, such as 50-500 IU pr. kg body weight.

In a still further aspect, the present invention relates to a pharmaceutical composition as described above comprising a combination of α-MSH or and/or α-MSH equivalent including the sequence His-Phe-Arg and recombinantly produced EPO optionally with a pharmaceutically acceptable carrier.

The pharmaceutical compositions according to the present invention may be prepared by use of conventional techniques known in the art and with conventional pharmaceutical carriers. Furthermore, the pharmaceutical composition may be in any form suitable for any of the uses as described herein.

Preferably, the pharmaceutical composition is in a form as described above.

### LEGEND TO FIGURES

Figure 1. Mean Arterial pressure before, during and after 45 minutes occlusion of the coliac trunk and the superior mesenteric artery in vehicle treated rats, rats treated with rh-EPO (200 U/kg body weight), rats treated with α-MSH (200 µg/kg body weight) or rats treated with the combination of rh-EPO (200 U/kg body weight) and α-MSH (200 µg/kg body weight).

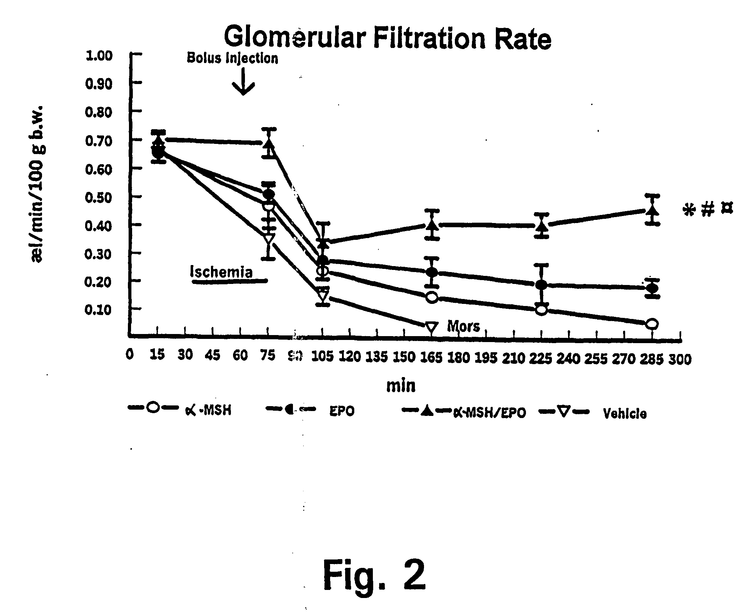

Figure 2. Glomerular filtration rate before, during and after 45 minutes occlusion of the coliac trunk and the superior mesenteric artery in vehicle treated rats, rats treated with rh-EPO (200 U/kg body weight), rats treated with α-MSH (200 µg/kg body weight) or rats treated with the combination of rh-EPO (200 U/kg body weight) and α-MSH (200 µg/kg body weight).

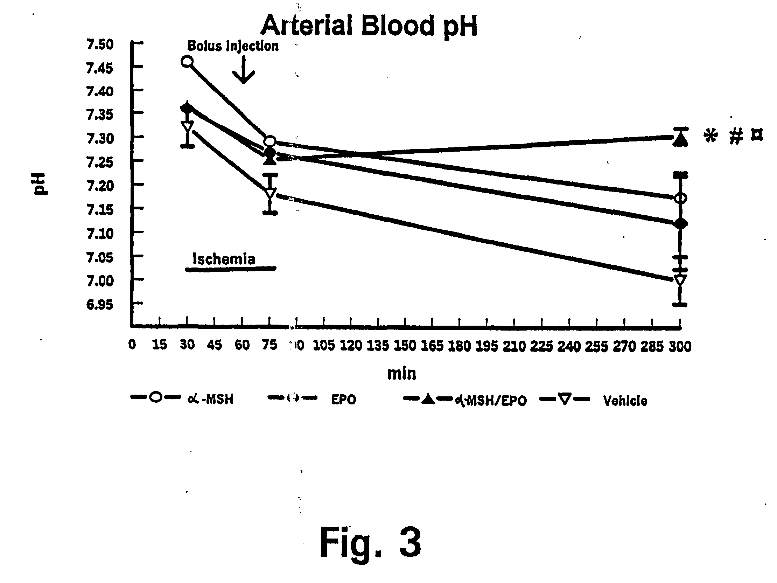

Figure 3. Arterial blood pH before, during and after 45 minutes occlusion of the coliac trunk and the superior mesenteric artery in vehicle treated rats, rats treated with rh-EPO (200 U/kg body weight), rats treated with α-MSH (200 µg/kg body weight) or rats treated with the combination of rh-EPO (200 U/kg body weight) and α-MSH (200 µg/kg body weight).

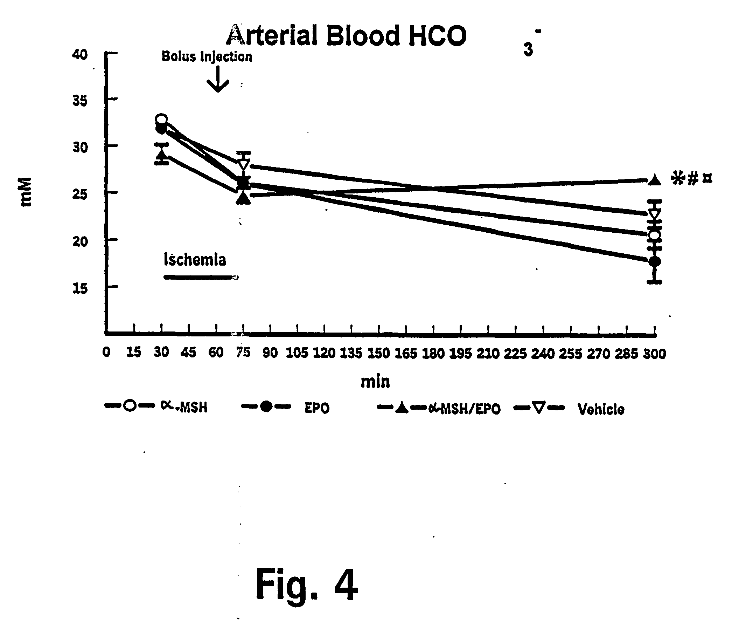

Figure 4. Arterial blood HCO₃⁻ before, during and after 45 minutes occlusion of the coliac trunk and the superior mesenteric artery in vehicle treated rats, rats treated with rh-EPO (200 U/kg body weight), rats treated with α-MSH (200 µg/kg body weight) or rats treated with the combination of rh-EPO (200 U/kg body weight) and α-MSH (200 µg/kg body weight).

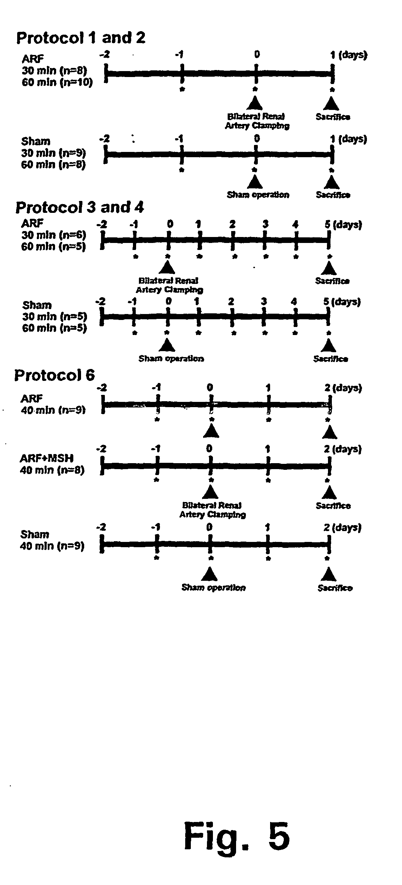

Fig 5. Diagram of the study design for testing of α-MSH in ischemia-induced acute renal failure (ARF).
   Protocol 1 and 2: ARF is established by temporary bilateral renal ischemia for 30 min or 60 min and monitored in the following 24 h; Sham operated rats matching ARF (30/1d) and ARF (60/1d).
   Protocol 3 and 4: ARF is established by temporary bilateral renal ischemia for 30 min or 60 min and monitored in the following 5 days; Sham operated rats matching ARF (30/5d) and ARF (60/5d).
   Protocol 6: ARF is established by temporary bilateral ischemia for 40 min and monitored in the following 2 days. The rats with ARF are divided into two groups: α-MSH non-treated and α-MSH treated; sham operated rats are treated with vehicle. The rats are maintained in the metabolic cages at the days marked with asterisk (*), allowing monitoring of urine excretion rates. Urine osmolality, creatinine, sodium and potassium were measured. Plasma is collected at the time of sacrifice for measurement of osmolality and concentration of sodium, potassium, creatinine.

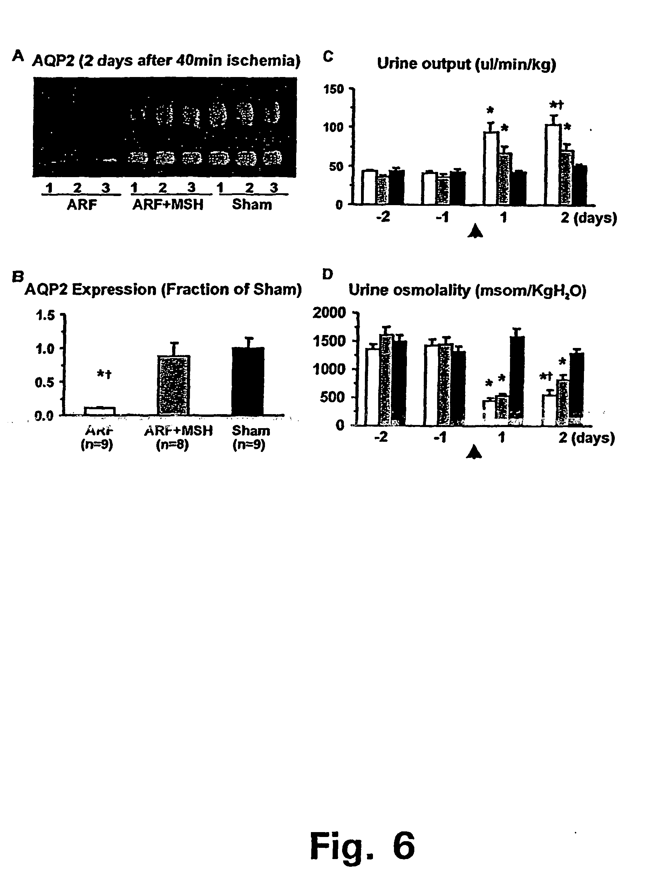

Fig 6. Effect of α-MSH treatment on AQP2 levels, changes in urine output and urine osmolality in rats with ARF (2 days after 40 min bilateral renal ischemia). A) The immunoblot is reacted with anti-AQP2. B) Densitometric analysis of all samples from ARF (40/2d), either in the absence of α-MSH treatment (ARF) or with α-MSH treatment (ARF+MSH) and sham operated rats. In the absence of α-MSH treatment, rats with ARF have a markedly decreased AQP2 expression levels (13 ± 3% of sham levels, P<0.05). AQP2 expression is 7 fold higher in response to α-MSH treatment of ARF rats compared to untreated rats. There is no difference in AQP2 expression between α-MSH treated ARF rats and sham operated rats. C) Time course of the changes in urine output and D) urine osmolality. Urine output is significantly increased and urine osmolality is significantly decreased after 40 min bilateral renal ischemia in ARF rats (both α-MSH treated and non-treated). ARF rats treated with α-MSH showed a reduced urine output and a higher urine osmolality compared with untreated ARF rats. **P* < 0.05, ARF are compared with sham operated rats. P < 0.05, α-MSH nontreated ARF rats are compared with α-MSH treated ARF rats.

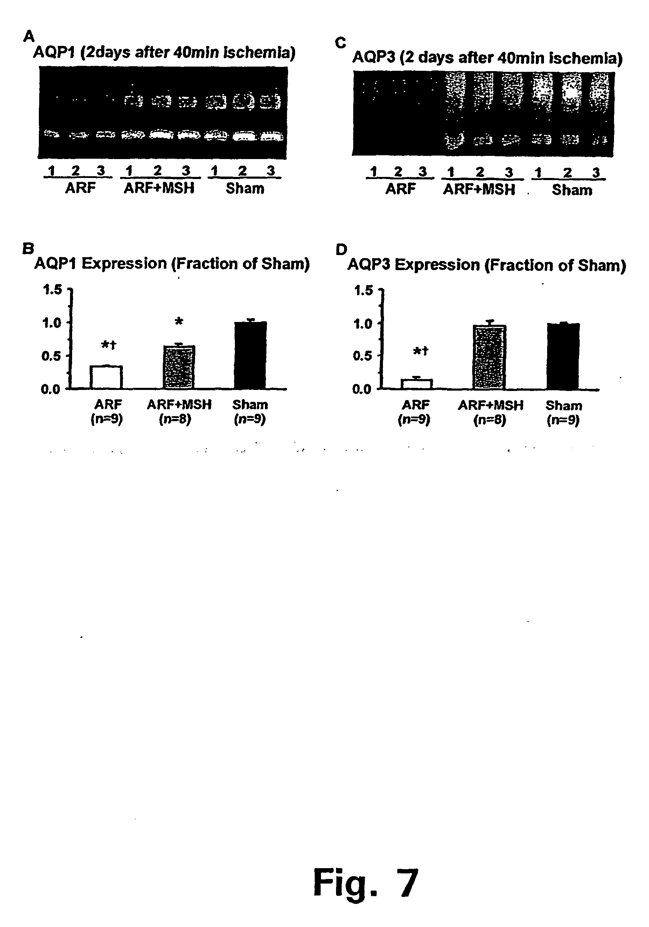

Fig 7. Effect of α-MSH treatment on AQP1 and AQP3 levels in rats with ARF (2 days after 40 min bilateral renal ischemia). A) The immunoblot is reacted with AQP1 immune serum. B) Densitometric analysis of all samples. C) The immunoblot is reacted with affinity purified anti-AQP3. D) Densitometric analysis of all samples. **P* < 0.05, ARF are compared with sham operated rats. *P* < 0.05, α-MSH nontreated ARF rats are compared with α-MSH treated ARF rats.

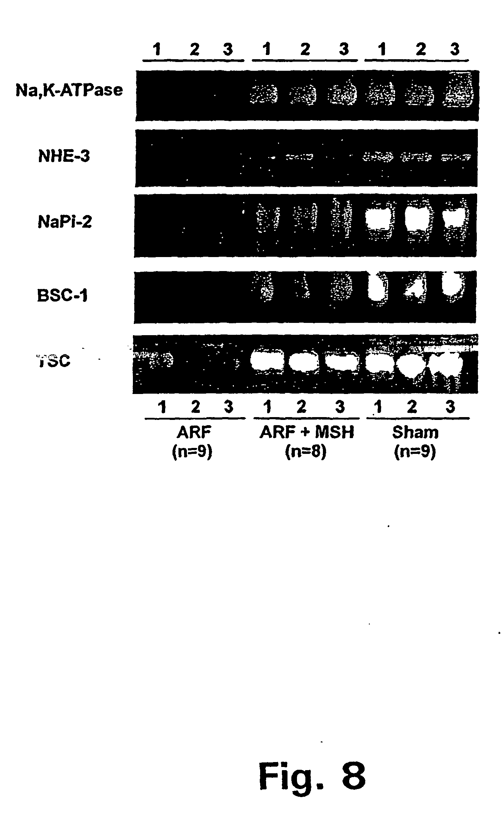

Fig 8. Effect of α-MSH treatment on the expression of several sodium transporters (Na,K-ATPase, NHE-3, NaPi-2, BSC-1 and TSC) in rats with ARF (2 days after 40 min bilateral renal ischemia). Semiquantitative immunoblotting demonstrates that α-MSH treatment during reperfusion in rats with ARF significantly reduce the decline in sodium transporter expressions following renal ischemia, compared to ARF rats which were not treated with α-MSH.

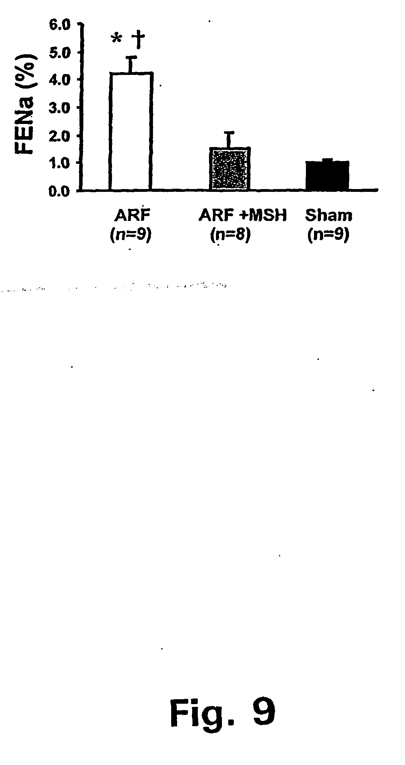

Fig 9. Effect of α-MSH treatment on FENa levels in rats with ARF (2 days after 40-min bilateral renal ischemia) and sham operated control rats. Rats with ARF have significantly increased FENa, two days after renal ischemia (4.2 ± 0.6%). In contrast, rats with ARF that were treated with α-MSH shows a markedly reduced FENa (1.5 ±0.6%), compared to rats with ARF that were not treated with α-MSH. The FENa in α-MSH treated rats with ARF is similar to that of sham operated rats (1 ± 0.1 %). **P* < 0.05, ARF rats are compared with sham operated rats. P < 0.05, ARF rats are compared with α-MSH treated ARF rats.

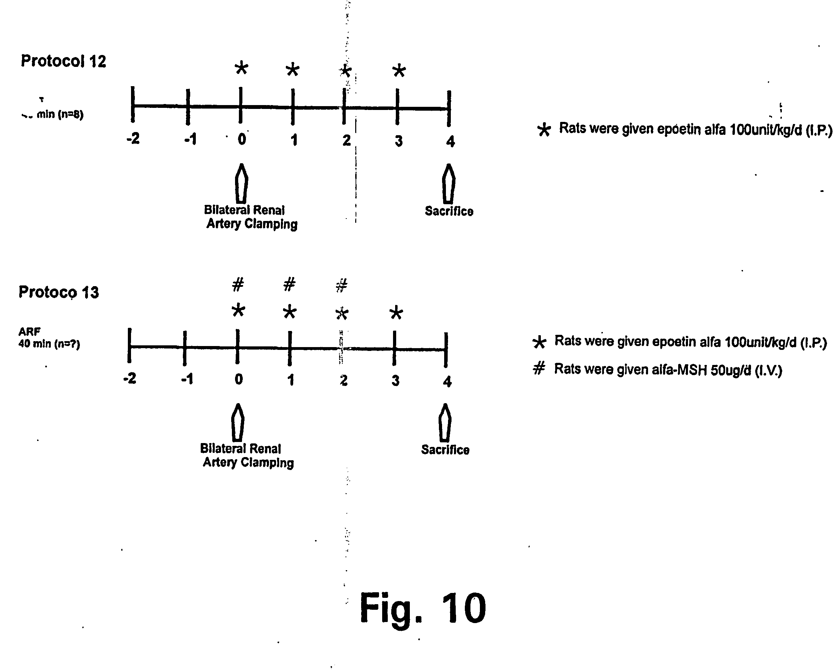

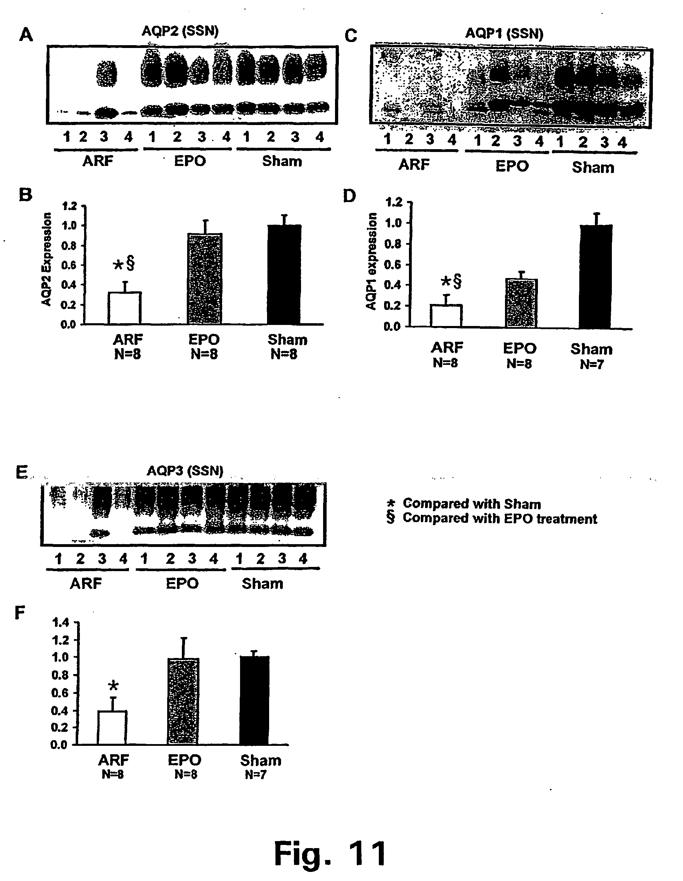

Fig 10. Protocols for experimental ischemia-induced acute renal failure (ARF) for testing the effect of treatment with EPO alone (i.e. erythropoetin; epoetin alfa), or combined treatment with both EPO and α-MSH.
Fig 11.
   A). Immunoblot (for AQP2) of membrane fractions from total kidney of rats (protocol 12) with ischemia-induced acute renal failure ARF that were subjected to vehicle treatment (ARF) or treated with EPO (EPO). Sham controls were included as well (Sham). EPO treatment prevented the downregulation of AQP2.
   B) Shows densitometric analysis.
   C) Immunoblot (for AQP1) of membrane fractions from total kidney of rats (protocol 12) with ischemia-induced acute renal failure ARF that were subjected to vehicle treatment (ARF) or treated with EPO (EPO). Sham controls were included as well (Sham). EPO treatment prevented the downregulation of AQP1.
   D) Shows densitometric analysis.
   E) Immunoblot (for AQP3) of membrane fractions from total kidney of rats (protocol 12) with ischemia-induced acute renal failure ARF that were subjected to vehicle treatment (ARF) or treated with EPO (EPO). Sham controls were included as well (Sham). EPO treatment prevented the downregulation of AQP3.
   F) Shows densitometric analysis.
Fig 12.
   A). Immunoblot (for AQP2) of membrane fractions from total kidney of rats (protocol 13) with ischemia-induced acute renal failure ARF that were subjected to vehicle treatment (ARF) or treated with EPO (EPO) plus α-MSH. Sham controls were included as well (Sham). Co-treatment with α-MSH and EPO prevented the downregulation of AQP2.
   B) Shows the densitometric analysis.
   C). Immunoblot (for AQP1) of membrane fractions from total kidney of rats (protocol 13) with ischemia-induced acute renal failure ARF that were subjected to vehicle treatment (ARF) or treated with EPO (EPO) plus α-MSH. Sham controls were included as well (Sham). Co-treatment with α-MSH and EPO prevented the downregulation of AQP1.
   D) Shows densitometric analysis.
   E). Immunoblot (for α-1-isoform of Na,K-ATPase) of membrane fractions from total kidney of rats (protocol 13) with ischemia-induced acute renal failure ARF that were subjected to vehicle treatment (ARF) or treated with EPO (EPO) plus α-MSH. Sham controls were included as well (Sham). Co-treatment with α-MSH and EPO prevented the downregulation of Na,K-ATPase.
   F) Shows densitometric analysis.

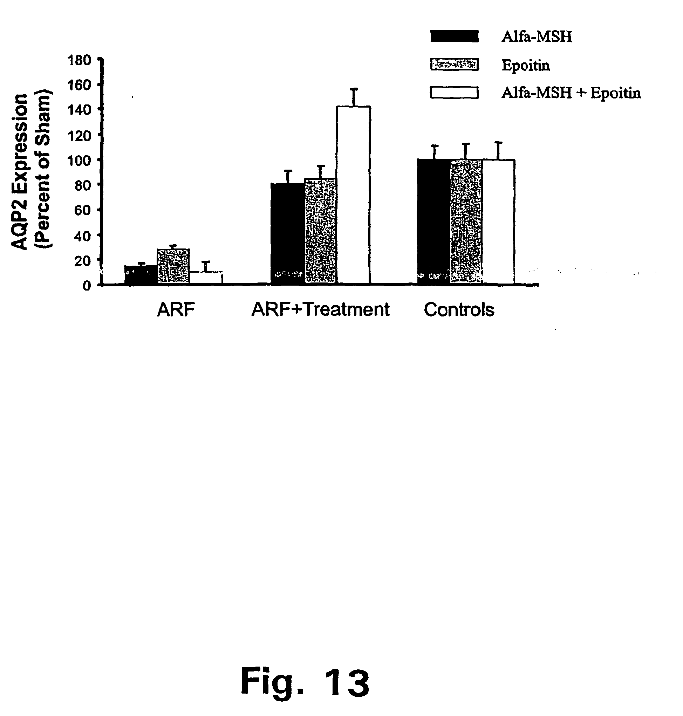

Figure 13. α-MSH treatment prevents downregulation of kidney AQP2 in ischemia-induced renal failure. EPO treatment prevents downregulation of kidney AQP2 in ischemia-induced renal failure. Combined treatment with EPO and α-MSH dramatically prevents downregulation of kidney AQP2 in ischemia-induced renal failure.

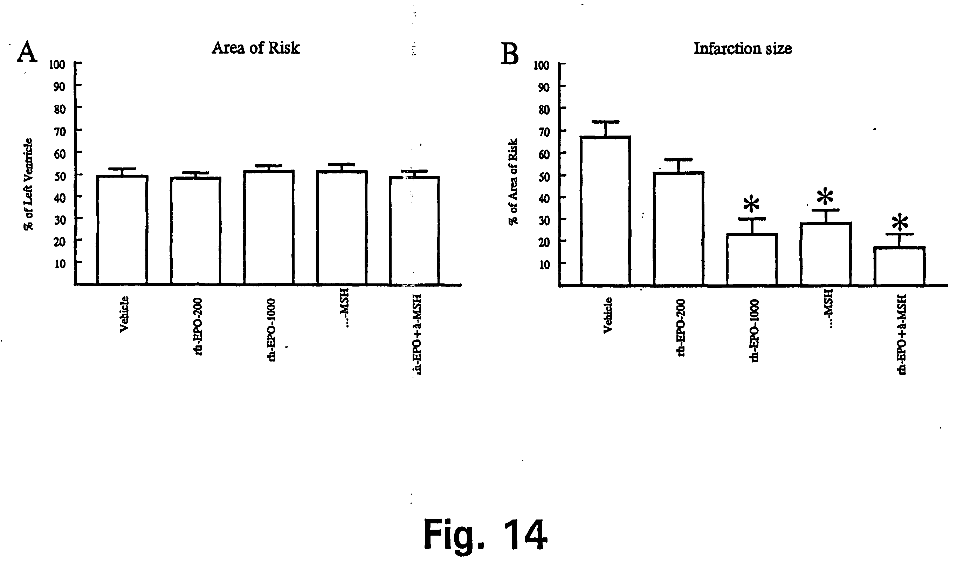

Figure 14. Myocardial ischemia was induced by sixty minutes occlusion of the left anterior descending artery in isoflurane anesthetised rats. After two hours reperfusion, the artery was reoccluded, evans blue injected i.v and the non-coloured area represented the occluded area (area of risk) (A). The area of risk (the ischemic area) was then isolated and incubated in a 0.5% triphenyltetrazolium chloride solution for 10 minutes at 37°C in order to quantitate the nechrotic part of the area of risk (B). *: P<0.05 vs. Vehicle treated animals.

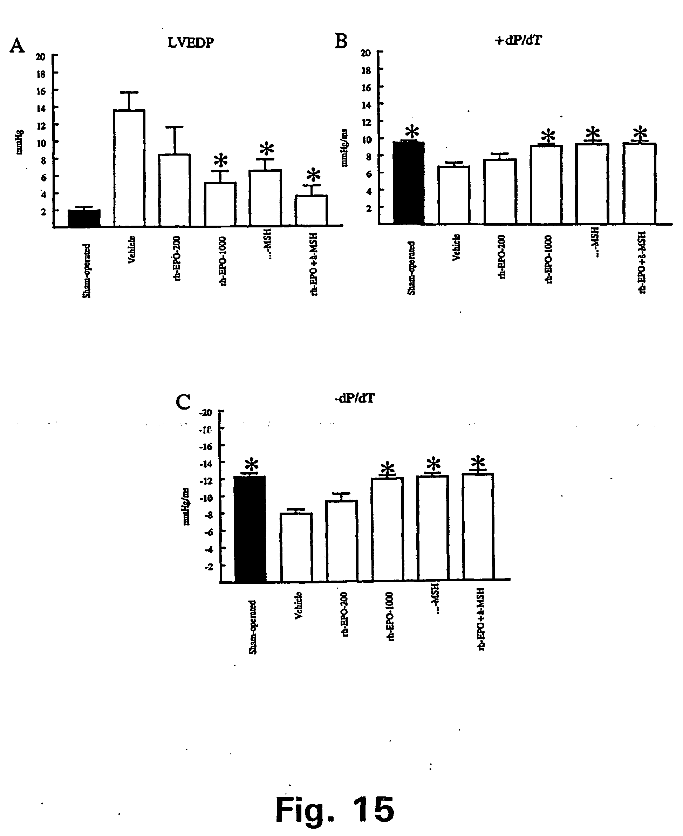

Figure 15. Measurement of left ventricular end diastolic pressure (LVEDP) (A), and positive (B) and negative (C) dP/dT in isoflurane anaesthetised rats three days after sixty minutes occlusion of the left anterior descending artery. *: P<0.05 vs. Vehicle treated animals.

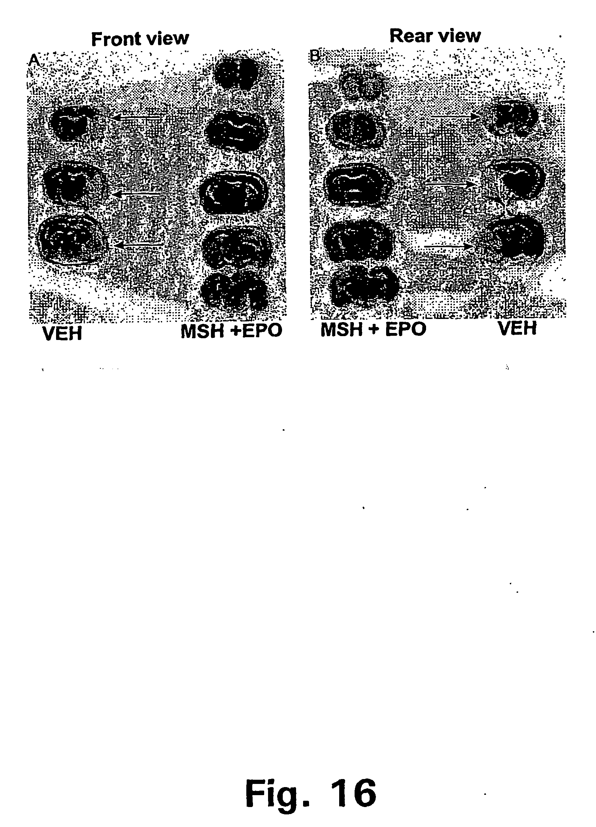

Figure 16. Brain slices subjected to supravital staining of living cells leaving dead cells in infarcted areas unstained (pale areas indicated by arrows). In the absence of treatment (i.e. treatment with vehicle alone (VEH) large areas of infarction is noted (arrows). In contrast treatment with α-MSH plus EPO (α-MSH+EPO) caused a significant decrease in the infarction size. The sections are displayed both from the front view and rear view.

### EXAMPLES

### Experimental animals

Barrier-bred and specific pathogen-free female Wistar rats (210-230 g) were obtained from the Department of Experimental Medicine, Panum Institute, University of Copenhagen, Denmark. The animals were housed in a temperature (22-24°C) and moisture (40-70%) controlled room with a 12-hour light-dark cycle (light on from 6:00 A.M. to 6:00 P.M.). All animals were given free access to tap water and a pelleted rat diet containing approximately 140 mmol/kg of sodium, 275 mmol/kg potassium and 23 % protein (Altromin catalogue no. 1310, Altromin International, Lage, Germany).

### Induction of intestinal ischemia in rats

Rats were anesthetized with halothane-nitrous oxide and permanent medical grade Tygon catheters were implanted into the abdominal aorta and into the inferior caval vein via a femoral artery and vein. Catheters were produced, fixed and sealed as described by Petersen et al. (J. Pharmacol. Exp. Ther. 258: 1-7, 1991). After instrumentation, the animals were housed individually. All surgical procedures were performed during aseptic conditions. To relieve postoperative pain, rats were treated with buprenorfin, 0.2 mg/kg body weight i.p. (Anorfin, GEA A/S, Copenhagen, Denmark), Two to three days later the rats were anesthetized with halothane-nitrous oxide and instrumented with a peruretral bladder catheter. The rats received constant i.v infusion of 150 mM Glucose with ³H-Inulin throughout, infusion rate 3.5 ml/hr. Then the coeliac trunk and the superior mesenteric artery were isolated near the aortic origins through a midline laparotomy. A 60 minutes calibration period was followed by a 30 minutes control period. Then the coeliac trunk and the superior mesenteric artery were clamped for 45 minutes followed by four hours reperfusion. Mean arterial blood pressure (MAP) and Heart Rate (HR) was followed throughout. Urine was collected in a 30 minutes control period, during the 45 minutes ischemia period and in four one-hour periods during reperfusion. Arterial blood samples for measurement of ³H-Inulin were collected at the end of each urine collection period. Arterial blood samples for measurements of pH and HCO₃⁻ were collected at the end of the control period, at the end of the ischemic period and at the end of the experiment. The body temperature was kept constant at 37° C throughout the experiment.

### Preventive effect of α-MSH optionally combined with EPO on ischemia-induced damage in the intestine.

The superior mesenteric artery and the coeliac trunk were clamped to produce a total occlusion of the arteries. After 45 minutes the clamps were removed and the intestine reperfused. The effect of i.v treatment with α-MSH or rh-EPO given alone or in combination in these rats was investigated. After reperfusion the untreated SAO-shock rats developed significant hypotension and all rats died within 4 hours from reperfusion. Rats treated with α-MSH (200 g/k g body weight) had a significantly attenuated decrease in systemic blood pressure and 80% of the rats were alive at the end of the 4 hour follow up period. Similarly, rats treated with rh-EPO (200 IU/kg body weight) had a significantly attenuated decrease in systemic blood pressure and 80% of the rats were alive at the end of the 4 hour follow up period. The combination of i.v α-MSH and i.v. erythropoietin treatment completly prevented death during the study period. Furthermore the fall in systemic blood pressure was significantly blunted compared to the untreated rats or the rats treated with either with α-MSH or rh-EPO.

### Experimental groups:

All the rats were subjected to ischemia/reperfusion. After 30 minutes ischemia the rats were subjected to one of the following i.v. treatments (N=6 in all groups):
Vehicle: 0.5 ml 150 ml Glucose
rh-EPO: 200 I.U. epoitin alfa (EPO)/kg body weight in 0.5 ml Glucose.
α-MSH: 200 µg α-melanocyte stimulating hormone (α-MSH)/kg body weight in 0.5 ml 150 ml Glucose.
α-MSH+rh-EPO: 200 µg α-MSH and 200 I.U.EPO/kg body weight in 0.5 ml 150 ml Glucose.

### Analytical procedures:

Urine volume was determined gravimetrically. Concentrations of sodium and ithium in plasma and urine were determined by atomic absorption spectrophotometry using a Perkin-Elmer (Allerød, Denmark) model 2380 atomic absorption spectrophotometer. ³H-Inulin in plasma and urine were determined by dual label liquid scintillation counting on a Packard Tri-Carp liquid scintillation analyser, model 2250CA (Packard Instruments, Greve, Denmark). Arterial blood pH and HCO₃ were measured by use of an ABL 555 (Radiometer, Copenhagen, Denmark).

### Statistics

Data are presented as mean ± S.E.. Within groups comparisons were analysed with Students paired t test. Between groups comparisons were performed by one way analysis of variance followed by Fishers Least Significant Difference test. Differences were considered significant at the 0.05 level.

### Survival

Table 1 summarizes survival rate and time in the four experimental groups. The vehicle treated rats all died within three hours after reperfusion. One of the animals treated with rh-EPO died 185 minutes after reperfusion and one of the α-MSH treated animals died 155 minutes after reperfusion. The rest survived the four hours reperfusion period. All rats that received combined treatment with rh-EPO and α-MSH survived throughout.

### Mean Arterial Pressure

Occlusion of the splanchnic arteries produced a significant increase in MAP in all rats. Reperfusion of the splanchnic arteries produced severe hypotension in the vehicle treated rats. This hypotensive response to reperfusion was significantly blunted in rats treated with rh-EPO or α-MSH. However, neither rh-EPO nor α-MSH could stabilize MAP at a level above the lower limit of renal perfusion pressure autoregulation (renal function, see later). Combined treatment with rh-EPO and α-MSH had a much more pronounced effect on MAP. The initial fall in MAP after reperfusion was almost completely abolished, and at the end of the 4 hours reperfusion period MAP was still above the lower limit of renal perfusion pressure autoregulation (84 ± 4 mmHG).

### Renal Function

Reperfusion of the splanchnic arteries significantly reduced glomerular filtration rate (GFR) in all groups. The reduction was most pronounced in the vehicle treated group with a complete stop in urine production two hours after reperfusion. Treatment with rh-EPO or α-MSH was unable to protect against further decreases in GFR during reperfusion, with the result that GFR was reduced by 71% in the rh-EPO and by 91% in α-MSH treated rats, respectively.

Combined treatment with rh-EPO and α-MSH significantly blunted the initial fall in GFR, and compared to treatment with either rh-EPO or α-MSH the combined treatment prevented any further decrease in GFR. In fact, the initial fall in GFR was partly reversed during the next four hours, and at the end of the experiment GFR was only reduced by 32% compared to the pre-SAO level.

### Acidosis

Occlusion/reperfusion of the splanchnic arteries produced severe acidosis in all rats. Treatment with rh-EPO, α-MSH or the combination of the two reduced the acidosis. However only the combination treatment was able to reverse the acidosis.

### Preventive effect of α-MSH optionally combined with EPO on ischemia-induced acute renal failure

### Experimental animals

Studies were performed on adult Munich-Wistar rats initially weighing 236 ± 6 gram (Møllegard Breeding centre Ltd., Eiby, Denmark). The rats were maintained on a standard rodent diet (Altromin, Lage, Germany) with free access to water.

### Induction of ischemia-induced acute renal failure in rats

After a period of acclimation to the metabolic cages, experimental ARF¹ was induced by occlusion of both renal arteries for 30 min or 60 min (Fig 5). During surgery, rats were anesthetized with halothane (Halocarbon Laboratories, NJ, USA), and were placed on a heated table to maintain rectal temperature at 37-38 °C. Both kidneys were exposed through flank incisions, mobilized by being dissected free from the perirenal fat. A small portion of the renal artery was gently dissected from the vein. The renal arteries were occluded with a smooth surfaced vascular clip (60 g pressure, World Precision Instruments, UK) for 30 min or 60 min. Total ischemia was confirmed by observing blanching of the entire kidney surface. During the period of ischemia, the wound was closed temporarily to keep body temperature. After the clips were
¹Animal experiments were carried out based on a licence from the Department of Justice, Denmark (Dyreforsøgstilsynet, Copenhagen, Denmark). removed, the kidneys were observed for additional 2-5 minutes to ensure color change indicating blood reflow and the wound was closed with 3-0 silk and surgical metal clamps. The rats were returned to metabolic cages, and daily 24 h urine output and water intake was measured according to the protocols depicted in Fig 5.

As a control group, rats were subjected to sham operations identical to the ones used for ARF rats without occlusion of both renal arteries. Sham operated rats were monitored in parallel with rats with ARF. All rats were killed under light halothane anesthesia and kidneys were rapidly removed and processed for membrane fractionation and immunoblotting at the same day.

### Clearance studies

The rats were maintained in the metabolic cages, allowing quantitative urine collections and measurements of water intake (Fig 5). Urine volume, osmolality, creatinine, sodium and potassium concentration were measured. Plasma was collected from abdominal aorta at the time of sacrifice for measurement of sodium and potassium concentration, creatinine, and osmolality.

### Experimental protocols

The following protocols were performed:
**Protocol 1:** 1) Rats with ARF established by bilateral renal ischemia for 30 minutes and monitored for additional 24 h (n= 8, ARF30/1d), 2) Sham operated rats (n=9) (Fig. 5).
**Protocol 2:** 1) Rats with ARF established by bilateral renal ischemia for 60 minutes and monitored for additional 24 h (n=10, ARF60/1d), 2) Sham operated rats (n=8) (Fig. 5).
**Protocol 3**: 1) Rats with ARF established by bilateral renal ischemia for 30 minutes and monitored for additional 5 days (n=6, ARF30/5d), 2) Sham operated rats (n=5) (Fig. 5).
**Protocol 4:** 1) Rats with ARF established by bilateral renal ischemia for 60 minutes and monitored for additional 5 days (n=5, ARF60/5d), 2) Sham operated rats (n=5) (Fig. 5).
**Protocol 5:** For immunocytochemistry, kidneys of rats with ARF (protocol identical to the one described in protocol 1 (n=4), 2(n=4), 3(n=3), and 4(n=3)) and of sham operated rats (protocol identical to protocol 1 (n=2), 2(n=2), 3(n=2), and 4(n=2)) were perfusion fixed (see below, not depicted in Fig 5).
**Protocol 6:** 1) rats with ARF established by bilateral ischemia for 40 min and monitored for additional 2 days. The animals-were divided in to two groups: α-MSH nontreated ARF (n= 9), α-MSH treated ARF (n=8). α-MSH (Phoenix Pharmaceutical Inc, Mountain View, CA, 50 g, i.v.) was given at the midpoint of the ischemic period, at 6 and 18 h postperfusion, and then every 24 h thereafter 2) Sham operated rats treated with vehicle (n=9) (Fig 5).
**Protocol 7:** For immunocytochemistry, kidneys of rats with ARF, either treated (n=5) with α-MSH or not treated (n=5) with α-MSH and of sham operated rats treated with vehicles (n=5) were perfusion fixed (see below, not depicted in Fig 5).
**Protocol 8:** Normal rats (n=5) were treated with α-MSH (Phoenix Pharmaceutical Inc, Mountain View, CA, 50 g, i.v.) using same protocol as protocol 6 (3 injections every 12 hours). Control rats (n=6) received only vehicle intravenously.
**Protocol 9:** 1) Rats with ARF established by bilateral ischemia for 40 min and monitored for additional 4 days. The animals were divided into two groups: EPO nontreated ARF (n= 8), EPO treated ARF (n=8). Epoetin was also called EPO or erythropoetin. Epoetin alfa (Jansen Pharmaceutical, 100 unit/kg/d I.P.) was given at the midpoint of the ischemic period and then every 24 h thereafter for 4 days. 2) Sham operated rats treated with vehicle (n=8) (Fig 10).
**Protocol 10:** 1) Rats with ARF established by bilateral ischemia for 40 min and monitored for additional 4 days. The animals were divided in to two groups: nontreated ARF (n= 4), α-MSH plus EPO treated ARF (n=4). α-MSH was given at the midpoint of the ischemic period, and then every 24 h thereafter for 4 days. EPO (epoetin alpha, Jansen Pharmaceutical, 100unit/kg/d I.P) was given simultaneously at the midpoint of the ischemic period and then every 24 h thereafter for 4 days. 2) Sham operated rats treated with vehicle (n=4) (Fig 10).
**Protocol 11.** Rats were treated with purimycin (50 or 180 mg/kg i.p., n=4 and 4) or adriamycin (7.5 mg/kg i.p.; n=4) and followed for 7 days and 21 days, respectively. Shams received saline i.p. (n=4).
**Protocol 12.** Left coronary artery ligation. Permantent ligation or ligation for two hours followed by reperfusion. The rats were either untreated or treated with i.v. α-MSH (50 µg/kg. body weight) once daily alone or in combination with erythropoeitin (100 U/kg body weight) once daily from the time of coronary artery ligation/reperfusion. N=8 in all groups.
Protocol 13. Occlusion of the major splanchnic arteries was followed by reperfusion in anesthetized rats. The superior mesenteric artery and the coeliac trunk were clamped to produce a total occlusion of the arteries. After 45 minuts the clamps are removed and the intestine reperfused. Then the rats were either untreated or treated with i.v. α-MSH (50 µg/kg. body weight) alone or in combination with erythropoeitin (100 U/kg body weight). Mean arterial pressure and mortality was followed for the next four hours. N=10 in all groups.

### Membrane fractionation for immunoblotting

The kidneys were homogenized (0.3 M sucrose, 25 mM imidazole, 1 mM EDTA, pH 7.2, containing 8.5 µM leupeptin, 1 mM phenylmethyl sulfonylfluoride) using an ultra-turrax T8 homogenizer (IKA Labortechnik, Germany), at maximum speed for 10 seconds and the homogenate was centrifuged in an Eppendorf centrifuge at 4,000 g for 15 minutes at 4°C to remove whole cells, nuclei and mitochondria. The supernatant was then centrifuged at 200,000 g for 1 hour to produce a pellet containing membrane fractions enriched for both plasma membranes and intracellular vesicles. Gel samples (Laemmli sample buffer containing 2% SDS) were made of this pellet.

### Electrophoresis and immunoblotting

Samples of membrane fractions from total kidney were run on 12% polyacrylamide minigels (BioRad Mini Protean II). For each gel, an identical get was run in parallel and subjected to coomassie staining to assure identical loading. The other gel was subjected to immunoblotting. After transfer by electroelution to nitrocellulose membranes, blots were blocked with 5% milk in PBS-T (80 mM Na₂HPO₄, 20 mM NaH₂PO₄, 100 mM NaCl, 0.1% Tween 20, pH 7.5) for 1 h, and incubated with antibodies to the following transporters: 1) AQP1 immune serum (LL266, diluted as1:2,000). 2) anti-AQP2 immune serum (LL127, diluted as 1:2,000). 3) affinity purified anti-AQP3 (LL178AP, 0.5 g/ml). 4)Na,K-ATPase (alpha-1 subunit). 5) NHE-3 (LL546AP). 6) NaPi-2 (LL696AP). 6) BSC-1 (LL320AP). 7) TSC (LL573AP). 8) Electrogenic Na,HCO3 cotransporter (rkNBC1). 9) Electroneutral Na,HCO3 cotransporter (NBC3). The labeling was visualized with horseradish peroxidase (HRP)-conjugated secondary antibody (P448, DAKO, Glostrup Denmark, diluted as 1:3,000) using enhanced chemiluminescence system (Amersham International, UK).

### Quantitation of kidney transporter density.

ECL films with bands within the linear range were scanned using an AGFA scanner (ARCUS II) and Corel Photopaint Software to control the scanner. The labeling density was quantitated of blots where samples from experimental kidneys were run on each gel with control kidneys from sham operated animals. The labeling density was corrected by densitometry of coomassie stained gels.

### Statistical analyses

Values were presented as means plus/minus standard errors. Comparisons between groups were made by unpaired t-test. P values < 0.05 were considered significant.

### Preparation of tissue for immunocytochemistry

The kidneys were fixed by retrograde perfusion via the abdominal aorta with periodate-lysine-paraformaldehyde (PLP: 0.01 M NalO₄, 0.075M lysine, 2% paraformaldehyde, in 0.0375 M Na₂HPO₄ buffer, pH 6.2). Kidneys were postfixed for 1 hour and tissue blocks were infiltrated for 30 min with 2.3 M sucrose containing 2% paraformaldehyde, mounted on holders and rapidly frozen in liquid nitrogen. For light microscopy, the frozen tissue blocks were cryosectioned (0.8 -1 µm) Reichert Ultracut S Cryoultramicrotome) and sections were incubated with the antibodies described above the labeling was visualized with HRP-conjugated secondary antibody (P448 or P447 1:100, DAKO, Glostrup, Denmark), followed by incubation with diaminobenzidine.

### Freeze-substitution of kidney tissue

The frozen samples were freeze-substituted in a Reichert Auto Freeze-substitution Unit (Reichert, Vienna, Austria). Briefly, the samples were sequentially equilibrated over 3 days in methanol containing 0.5% uranyl acetate at temperatures gradually increasing from -80°C to -70°C, and then rinsed in pure methanol for 24 h while increasing the temperature from -70°C to -45°C. At -45°C the samples were infiltrated with Lowicryl HM20 and methanol 1:1, 2:1 and, finally, pure Lowicryl HM20 before UV-polymerization for 2 days at -45°C and 2 days at 0°C. For electron microscopy immunolabeling was performed on ultrathin Lowicryl HM20 sections (60-80 nm), which were incubated overnight at 4°C with antibodies diluted in PBS with 0.1 % BSA or 0.1% skimmed milk. The labeling was visualized with goat-anti-rabbit IgG conjugated to 10 nm colloidal gold particles (GAR.EM10, BioCell Research Laboratories, Cardiff, UK) diluted 1:50 in PBS with 0.1 % BSA. The sections were stained with uranyl acetate and lead citrate before examination in Philips CM100 or Philips 208 electron microscopes.

### Results

### α-MSH treatment reduces the ischemia-induced defects in urinary

### concentration

ARF for 30, 40 or 60 min followed by release of arterial ligation for 1 or 5 days is associated with severe downregulation of aquaporins and sodium transporters (Table 1, 2 and 5; protocol 1-6) associated with marked changes in renal water and sodium excretion and urinary concentration (Tables 4-5). α-MSH, which is known to inhibit both inflammatory and nitric oxide pathways, has recently been demonstrated by Star and his colleagues to be effective in reducing the ischemia/reperfusion injury in rats (Chiao, H et al. J.Clin.Invest. 99: 1165-1172, 1997.) This has been suggested to be ascribed, in part, to reduced neutrophil migration and inhibited production of neutrophil chemokines (Chiao, H et al. J.Clin.Invest. 99: 1165-1172, 1997.) We therefore tested whether α-MSH treatment affects the expression of aquaporins and the changes in the urinary concentration in postischemic ARF, with the same protocols described by Star and his colleagues (Chiao. H et al. J.Clin.invest. 99 1165-1172 1997.). Rats with ARF (ARF40/2d, protocol 6) showed significant acute renal insufficiency, compared with sham operated rats (Table 4). α-MSH treatment markedly reduced the severity of renal insufficiency since ARF rats which were not treated with α-MSH demonstrated significantly higher plasma creatinine levels (194 ± 45 µmol/L, P < 0.05), compared with ARF rats treated with α-MSH (68 ±15 µmol/L). Thus, α-MSH markedly inhibits the decline in renal functions in response to renal ischemia and reperfusion, consistent with previous observations (1). Furthermore, α-MSH treatment significantly reduced the degree of polyuria which was encountered in the postischemic period (Table 4, Fig 6C). Consistent with this, urine osmolality, U/P osm, and TcH₂O in α-MSH treated ARF rats were significantly improved, compared with α-MSH non-treated ARF rats (Table 4, Fig 6D).

### α-MSH treatment reduces the ischemia-induced downregulation of renal aquaporins

Fig 6 shows the effects of α-MSH treatment on the kidney AQP2 levels, with changes in urine output and urine osmolality in ARF and sham operated rats. Rats with ARF, which were not treated with α-MSH during reperfusion period demonstrated markedly decreased AQP2 levels (13 ± 3% of sham levels, P<0.05) compared with sham operated rats (100 ± 15%, Fig 6A and B). Importantly, α-MSH-treatment prevented the downregulation of AQP2. ARF rats treated with α-MSH had almost 7 fold higher AQP2 expression levels compared to untreated ARF rats (Fig 6A and B).

Next it was examined if the downregulation of AQP3 and AQP1 was also prevented by α-MSH treatment. Similar to the observations on AQP2 expression (Fig 6A) AQP3 expression was also approximately 7 fold higher in the α-MSH treated rats compared to untreated rats (Fig 7) and this expression was not different from the levels in Sham operated rats. Also AQP1 expression was markedly higher (2 fold) in the α-MSH treated ARF rats compared to untreated ARF rats (Fig 7). This supports the view that reduced expression of aquaporins may play a role in the functional defects demonstrated to be associated with experimental ischemia-induced ARF. Control experiments with normal rats treated with α-MSH did not show any changes in urine output and urine osmolality.

### Immunocytochemistry confirms that α-MSH treatment has protective effects in response to renal ischemia and reperfusion

Immunocytochemistry confirmed that there was an overall decrease in AQP2 expression in inner medullary collecting duct principal cells from ARF rats (40/2d) without α-MSH treatment, compared with α-MSH treated ARF rats or sham operated control rats. The marked reduction in AQP2 labeling in inner medulla was consistent with a significant decreased density observed by immunoblotting (Fig 6A and B). The part of the remaining labeling was associated with apical plasma membrane domains of collecting duct principal cells. In α-MSH treated ARF rats, AQP2 labeling was unchanged compared to sham operated rats, demonstrating α-MSH treatment inhibited the decreased expression of AQP2 in response to renal ischemia and reperfusion injury. The labeling of AQP3 and AQP4 in inner medullary collecting duct from α-MSH treated ARF rats were also unchanged. This confirmed that α-MSH treatment during reperfusion period after renal ischemic insults is effective in inhibiting decreased expression of aquaporins in the inner medullary collecting duct.

### α-MSH treatment markedly inhibits the decline in renal functions following renal ischemia and reperfusion

α-MSH treatment markedly reduced the severity of acute renal insufficiency since ARF rats treated with α-MSH had significantly lower plasma creatinine levels (68 ±15 µmol/L, P < 0.05), compared with ARF rats which were not treated with α-MSH (194 ± 45 µmol/L (Table 5). Moreover, α-MSH treatment significantly reduced the increased FENa (4.2 ± 0.6% in ARF rats vs 1.5 ± 0.6% in α-MSH treated ARF rats, P<0.05, Fig 5), and the degree of polyuria (Table 5). Consistent with this, urine osmolality, U/P osm, and TcH₂O in α-MSH treated ARF rats were also significantly improved (Table 5). Thus, α-MSH significantly inhibits the changes in renal sodium handling following renal ischemia and reperfusion.

### α-MSH treatment reduces the ischemia-induced decrease in sodium transporters in ischemia-induced ARF

As shown in Fig 8 and Table 6, α-MSH treatment dramatically prevents the decrease in the abundance of all the investigated sodium transporters following renal ischemia and reperfusion. This is consistent with marked improvement in renal sodium handling in response to α-MSH treatment of rats with acute renal failure (Fig 9, Table 5). In contrast, in rats with ARF (40/2d) which were not treated with α-MSH, the abundance of all the major sodium transporters investigated were significantly reduced in post-ischemic kidney compared to sham controls. Immunocytochemistry also revealed that α-MSH treatment during reperfusion after renal ischemia is effective in inhibiting the downregulation of sodium transporter expression in post-ischemic kidneys.

### Treatment with EPO prevents reduction in renal function and downregulation of renal transporters in ischemia-induced acute renal failure.

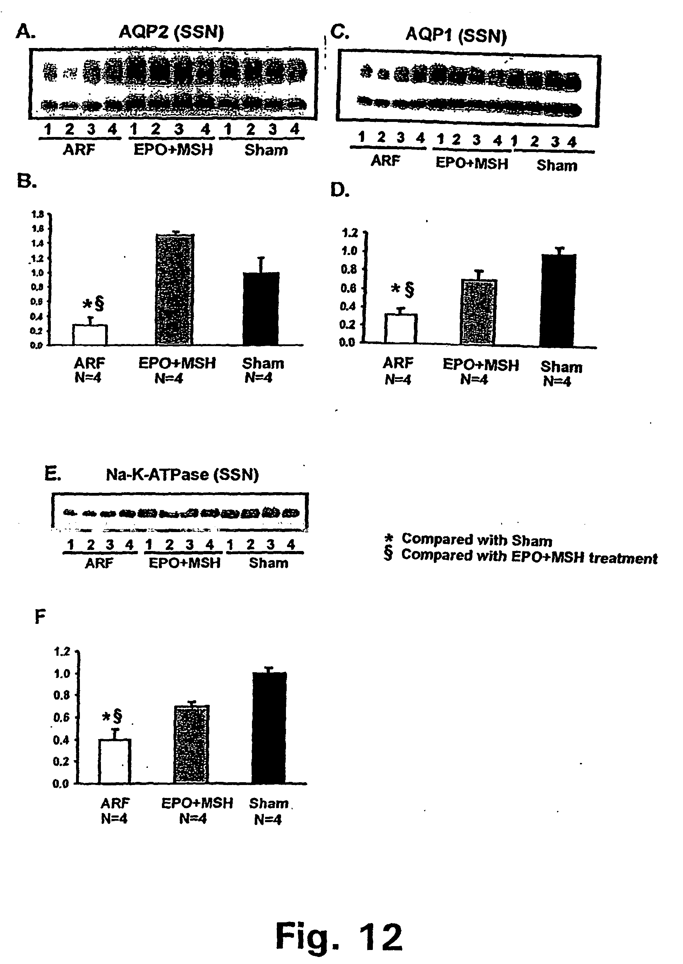

Rats with ARF established by bilateral ischemia for 40 min and were monitored for additional 4 days and EPO (protocol 12) was given at the time of the ischemic insult and then every 24 hours (Fig 10). As shown in Figure 12 EPO treatment dramatically reduce the downregulation of AQP1, AQP2 and AQP3 expression demonstrating a clear effect.

### Co-treatment with EPO and α-MSH prevents reduction in renal function and downregulation of renal transporters in ischemia-induced acute renal failure.

As shown in Figur 12 co-treatment with EPO and α-MSH dramatically reduce the downregulation of AQP1, AQP2 and Na,K-ATPase expression demonstrating a clear effect. Functional parameters regarding the urine production demonstrated an effect already at day 1 indicating that co-treatment with both EPO and α-MSH is more effective than treatment with EPO alone.

### Preventive effect of α-MSH optionally combined with EPO on myocardial ischemia

### Experimental animals

Barrier-bred and specific pathogen-free female Wistar rats (250 g) were obtained from the Department of Experimental Medicine, Panum Institute, University of Copenhagen, Denmark. The animals were housed in a temperature (22-24°C) and moisture (40-70%) controlled room with a 12-hour light-dark cycle (light on from 6:00 A.M. to 6:00 PM). All animals were given free access to tap water and a pelleted rat diet containing approximately 140 mmol/kg of sodium, 275 mmol/kg potassium and 23 % protein (Altromin catalogue no. 1310, Altromin International, Lage, Germany).

### Animal preparation

Rats were anaesthetized in an inhalation chamber with 4% isoflurane in O₂. After insertion of an endotracheal tube, the animal was artificially ventilated with1.0 % isoflurane in O₂ using of Hugo Basile Rodent ventilator. Tidal volume was 8-10 ml/kg body weight and respiratory rate 75 min⁻¹ which maintained arterial pH between 7.35 and 7.45. During surgery the animal was placed on a heated table that maintained rectal temperature at 37-38°C. Permanent medical grade Tygon catheters were implanted into the inferior caval vein and into the abdominal aorta via the femoral vein and artery. Standard ECG (second lead) was measured using a Hugo Sachs ECG Coupler and collected on line at 4,000 Hz in PowerLab. After parasternal thoracotomy and opening of the pericardium, the left anterior descending coronary artery (LAD) was localized visually. Rats where the LAD could not be visualized were used as sham-operated control rats. An atraumatic 6-0 silk suture with an occluder that allowed reopening of the ligature, was placed around the LAD between the pulmonary trunk and the lower right end of the left auricle. After 10 minutes, the left anterior descending coronary artery (LAD) was occluded. Successful occluding was confirmed by alterations in ECG (ST-segment elevation and increase in R-wave amplitude) and by fall in MAP. Reperfusion was made after 60 minutes by opening the occluder. Control rats were sham-operated.

### Experimental groups:

The rats were subjected to one of the following i.v treatments:
**Vehicle:** 0.5 ml 150 mM NaCl once daily.
**α-MSH:** 200 µg α-melanocyte stimulating hormone/kg body weight in 0.5 ml 150 mM NaCl once daily.
**rh-EPO-200:** 200 I.U. epoitin alfa/kg body weight in 0.5 ml 150 mM NaCl once daily.
**rh-EPO-1000:** 1000 I.U. epoitin alfa/kg body weight in 0.5 ml 150 mM NaCl once daily.
**α-MSH+rh-EPO:** 200 µg α-MSH/kg body weight and 200 I.U. EPO/kg body weight in 0.5 ml 150 mM NaCl once daily.
**Control:** Sham operated rats treated with vehicle (0.5 ml 150 mM NaCl) once daily.
The first dose was given after 30 minutes ischemia.

### Myocardial infarction/ ischemia (coronary artery ligation and reperfusion) in rats

Ligation of the anterior intraventricular ramus from the left coronary artery (LCAL) in rats induce ischemia and eventually infarction in the anterior wall of the left ventricle of the heart. The infarction of the heart is associated with the development of heart failure characterized by a significantly impaired diastolic function evaluated through an elevated end diatolic pressure in the left ventricle (LVEDP) and will eventually result in the formation of edema and increased mortalility. Rats were anaesthetized placed on a heated table that maintained rectal temperature at 37-38 C, and ventilated though an endotracheal tube. Tidal volume and respiratory rate was adjusted to maintain arterial pH between 7.35-7.45. A parasternal thoracotomy was performed, and a 6.0 silk suture was placed between the pulmonary trunk and the left auricle where the intraventricular ramus of the left coronary artery is placed. The suture was ligated and the intraventricular ramus thereby ligated. This procedure induces total ischemia in a part of the anterior wall of the left ventricle. In one series of animals the ligation was permanent. In another series of rats, the ligation was removed after two hours in order to induce reperfusion. Only data from rats that survived the surgical intervention is presented.

### Myocardial infarction: preventive effect of ischemia-induced damages by α-MSH or EPO treatment.

The effect of α-MSH treatment alone or in combination with erythropoeitin on the remodulation of the ischemic area in rats with LCAL was investigated. Measurement of LVEDP on day four after LCAL, both in rats with permanent ligation and rats with reperfusion after two hours ischemia showed that α-MSH treatment given alone or in combination with erythropoeitin significantly reduced LVEDP compared to untreated rats. Morphological examination revealed that α-MSH treatment significantly modulated the ischemic area with the result that the size of the infaction was decreased compared to rats without treatment. The reduction in infaction size was even more pronounced when α-MSH was given in combination with erythropoeitin. Together these results indicate that α-MSH treatment alone or in combination with erythropoeitin have profound beneficial effects on the modulation of the ischemic area in the myocardium in rats with experimental induced myocardial ischemia.

### Protocol 14: Determination of the size of the ischemic and necrotic myocardium.

The rats were kept anaesthetized after the ischemia/reperfusion and re-occluding of the LAD was performed after two hours reperfusion. During this period, ECG and MAP were measured continuously. Then Evans Blue dye (1 ml; 2% w/v) was administered i.v. to determine the size of the ischemic area. The heart was removed and cut into horizontal slices to determine the size of the ischemic area and to separate the ischemic myocardium from the non-ischemic myocardium. The ischemic area was isolated and incubated in a 0.5% triphenyltetrazolium chloride solution for 10 minutes at 37°C. The size of the necrotic tissue was then measured gravimetrically. N=6 in all groups.

### Protocol 15: Measurement of hemodynamic dysfunction three days after ischemia/reperfusion.

After ischemia/reperfusion and initial treatment with vehicle, α-MSH, rh-EPO or α-MSH+EPO, the rats were placed in separate cages and were given free access to tap water and standard rat diet. To relieve postoperative pain, rats were treated with buprenorfin, 0.2 mg/kg body weight i.p. twice daily for two days. Three days after ischemia/repserfusion the rats were anaesthetized with isoflurane in O₂. The concentration of isoflurane was adjusted to maintain after-load during anaesthesia, i.e MAP was stabilized at 95-100 mmHg. A 2F Millar microtip catheter was inserted into the left ventricle via the right carotic artery for measurement of left ventricular end diastolic pressure (LVEDP), and positive and negative dP/dT. Standard ECG (second lead) and MAP were measured throughout. The development of Q-waves was used as a sign of transmural infarction. N=6 in all groups, except in the Vehicle and the rh-EPO-200 groups, where the number of animals due to sudden death during the 3 days study period were 11 and 8 respectively.

### Statistics

Data is presented as mean ± S.E.. Within-group comparisons were analyzed with Student's paired *t* test. Between-group comparisons were performed by one way analysis of variance followed by Fishers Least Significant Difference test. Differences were considered significant at the 0.05 level.

### Mortalility and Infaction size

All animals presented in study protocol 14 survived the two hours reperfusion period. In study protocol 15, the rats were followed for three days and during this period the mortality in the vehicle treated group was 45% (5 out of 11 animals) and 25% in the rats treated with low dose rh-EPO (2 out of 8 animals). All animals survived in the other groups, i.e. high dose rh-EPO treatment, α-MSH treatment; combined treatment with low dose rh-EPO and α-MSH, and in the sham-operated controls.

The mean area of risk was similar in all ischemia/reperfusion groups when the hearts were examined two days after reperfusion (figure 12). Vehicle treated rats had an infarct size of 67±7% of the area of risk. Low dose rh-EPO did not significantly reduce the size of the infarcted part of the area of risk. However, high dose rh-EPO as well as α-MSH significantly reduced infarction size. High dose Rh-EPO reduced the infarction size by 65% and α-MSH reduced the infarction size by 58% compared to the vehicle treated animals. Combined treatment with low dose rh-EPO and α-MSH reduced the size of the infarction size by 75%. The sham-operated time-control animals had no infarction (data not shown).

### Changes in ECG

Baseline hemodynamics and ECG were similar in all groups (data not shown). Ischemia induced ST-elevation (and hypotension) in all animals. Sham-operation caused no changes in ECG (or MAP).

The surviving rats treated with vehicle and low dose rh-EPO developed coronary Q-waves when examined three days after ischemia/reperfusion. This sign of transmural myocardial infarction was lagging in rats treated with high dose rh-EPO, α-MSH and combined treatment with low dose rh-EPO and α-MSH.

### Cardiac function three days after ischemia/reperfusion

At day three, the surviving vehicle treated animals had significantly increased LVEDP compared to Sham-operated time-controls (figure 14). +dP/dT was significantly decreased arid -dP/dT increased in the vehicle treated animals. Low dose rh-EPO did not prevent the impairment in cardiac function found in the vehicle treated animals. However, the impairment in cardiac function was significantly blunted in rats treated with high dose rh-EPO, α-MSH or combined treatment with low dose rh-EPO and α-MSH.

### Combined Treatment with rh-EPO and α-MSH dramatically prevent brain infarct in response to temporarily occlusion of blood supply.

We examined the effect of combined treatment with recombinant human erythropoietin (rh-EPO) (200 U/kg body weight) and α-Melanocyte Stimulating Hormone (α-MSH) (200 µg/kg body weight) on brain infarction in a rats model of apoplexia.

### Methods:

Transient focal cerebral ischemia was induced in Wistar rats, using the intraluminal occlusion model of the median cerebral artery (MCA) for two hours followed by reperfusion, by the withdrawal of the suture. After one hour of occlusion animals were either treated with α-MSH and epoetin (combined treatment) or with vehicle. The animals were sacrificed 48 hours following reperfusion, brains were removed, sectioned in 2 mm thick slices and stained with the TTC method, (by incubating the slices in 1 % TTC solution at 37 Celsius for 30 min) to demarcate the ischemic area. High-resolution images of the slices were obtained using a flat-bed scanner.

### Results:

As shown in Fig 15 combined treatment with α-MSH and epoetin dramatically prevented brain infarction leaving only very minor areas with infarction. Thus this treatment procedure provide an unprecedented prevention of infarction associated with arrest in brain blood supply.

### TABLES

**Table 1**

| Effects of vehicle (150 mM glucose); rh-EPO (200 U/kg body weight); αMSH (200 µg/kg body weight) and the combination of rh-EPO (200 U/kg body weight) and αMSH (200 µg/kg body weight) on survival in splanchnic artery occlusion shocked rats. | | |
|---|---|---|
| | Animals surviving the 240 minuts reperfusion period | 240 minuts survival rate % |
| Vehicle: | 0/6 | 0 |
| rh-EPO: | 5/6 | 83* |
| α-MSH: | 5/6 | 83* |
| α-MSH+rh-EPO: | 6/6 | 100*#¤ |

| | | |
|---|---|---|
| *: different from Vehicle treated rats; p<0.05; #: different from rh-EPO treated rats; P<0.05; ¤: different from α-MSH treated rats; p<0.05. | | |

**Table 2. Changes in expression of AQP1, AQP2, and AQP3 in ischemia-induced ARF rats.**

| | **ARF(30 min/1 day) (protocol 1)** | **ARF(60min/1 day) (protocol 2)** | **ARF(60min/5 days) (protocol 4)** |
|---|---|---|---|
| ***AQP1*** | | | |
| ARF | 30 ± 5%* (n=8) | 51 ± 5%* (n=10) | 6 ± 2%* (n=5) |
| Sham | 100 ± 15% (n=9) | 100 ± 14% (n=8) | 100±13% (n=5) |

| ***AQP2*** | | | |
|---|---|---|---|
| ARF | 40 ± 11%*(n=8) | 31 ± 9%* (n=10) | 11 ± 4%* (n=5) |
| Sham | 100 ± 7% (n=9) | 100 ± 9%(n=8) | 100 ± 14%(n=5) |

| ***AQP3*** | | | |
|---|---|---|---|
| **ARF** | 53 ± 18%* (n=8) | 21 ± 4%* (n=10) | 9 ± 4%* (n=5) |
| Sham | 100 ± 22% (n=9) | 100 ± 17% (n=8) | 100 ± 18% (n=5) |

| | | | |
|---|---|---|---|
| Values are expressed as means ± SE. **P* < 0.05 when ARF rats were compared with sham operated rats. | | | |

**Table 3. Changes in the expression levels of major sodium transporters in rats with ischemia-induced ARF**

| | **ARF (30/1d)** | **ARF(30/5d)** | **ARF(60/1d)** | **ARF(60/5d)** |
|---|---|---|---|---|
| ***Na,K-ATPase*** | | | | |
| ARF | 51 ± 11 %*(n=8) | 69 ± 18% (n=6) | 22 ± 8%*(n=10) | 14 ± 7%*(n=5) |
| Sham | 100 ± 5% (n=9) | 100 ± 19% (n=5) | 100 ± 10% (n=8) | 100 ± 13% (n=5) |

| ***NHE-3*** | | | | |
|---|---|---|---|---|
| ARF | 28 ± 6%*(n=8) | 68 ± 19% (n=6) | 20 ± 8% (n=10) | 2 ± 1%* (n=5) |
| Sham | 100 ± 9%(n=9) | 100 ± 23% (n=5) | 100 ± 19% (n=8) | 100 ± 16% (n=5) |

| ***NaPi-2*** | | | | |
|---|---|---|---|---|
| ARF | 14 ± 6%*(n=8) | 67 ± 16% (n=6) | 19 ± 10%*(n=10) | 2 ± 0.8%*(n=5) |
| Sham | 100 ± 13% (n=9) | 100 ± 20% (n=5) | 100 ± 3% (n=8) | 100 ± 20%(n=5) |

| ***BSC-1*** | | | | |
|---|---|---|---|---|
| ARF | 68 ± 12% (n=8) | 72 ± 8% (n=6) | 11 ± 3%*(n=10) | 7 ± 2%*(n=5) |
| Sham | 100 ± 11% (n=9) | 100 ± 14% (n=5) | 100 ± 9% (n=8) | 100 ± 6% (n=5) |

| ***TSC*** | | | | |
|---|---|---|---|---|
| ARF | 91 ± 19% (n=8) | 80 ± 17% (n=6) | 17 ± 5%*(n=10) | 11 ± 7%*(n=5) |
| Sham | 100 ± 5% (n=9) | 100 ± 5% (n=5) | 100 ± 10% (n=8) | 100 ± 1% (n=5) |

| | | | | |
|---|---|---|---|---|
| For densitometry of immunoblots, sodium transporter labeling was calculated as a fraction of the mean value from sham operated controls. **P* < 0.05 when ARF rats were compared with sham operated rats. | | | | |

**Table 4. Urine output and urinary concentrating ability two days after release of 40 minutes bilateral renal ischemia with or without alpha-MSH treatment**

| | **ARF (n=9)** | **ARF + MSH (n=8)** | **Sham (n=9)** |
|---|---|---|---|
| ***p-creat (µmol*/*L)*** | 194 ± 45* | 68 ± 15* | 32 ± 1 |
| ***Ccr (ml*/*min)*** | 0.4 ± 0.1 *' | 0.8 ± 0,1* | 1.3 ± 0.06 |
| ***Plasma urea nitrogen (mmol*/*L)*** | 36 ± 5*' | 12 ± 3* | 3.8 ± 0.3 |

| *Urine output (µl*/*min*/*Kg)* | | | |
|---|---|---|---|
| Baseline period | | | |
| Day-2 | 44 ± 1 | 35 ± 3 | 44 ± 4 |
| Day-1 | 40 ± 3 | 36 ± 4 | 42 ± 4 |
| After operation | | | |
| Day 1 | 94 ± 13* | 67 ± 9* | 42 ± 3 |
| Day 2 | 104 ± 12* | 71 ± 8* | 51 ± 2 |

| ***Urine osmolality (mosm*/*KgH₂O)*** | | | |
|---|---|---|---|
| Baseline period | | | |
| Day - 2 | 1356 ± 94 | 1602 ± 150 | 1503 ± 96 |
| Day - 1 | 1435 ± 99 | 1439 ± 135 | 1315 ± 97 |

| After operation | | | |
|---|---|---|---|
| Day 1 | 443 ± 51* | 530 ± 49* | 1587±138 |
| Day 2 | 557 ± 89* | 824 ± 80* | 1297 ± 74 |
| ***U*/*P osm**** | 1.7 ± 0.3* | 2.7 ± 0.3* | 4.3 ± 0.3 |
| ***TcH₂O (µl*/*min*/*Kg)**** | 51 ± 6* | 118 ± 18* | 163 ± 10 |

| | | | |
|---|---|---|---|
| **P* < 0.05 when ARF rats, which were either treated or not treated with α-MSH, were compared with sham operated rats. '*P* < 0.05 when ARF rats not treated with α-MSH were compared with ARF rats treated with α-MSH. * U/P osm and TcH₂O were measured at Day 2. | | | |

**Table 5. Changes in renal function two days after release of 40 minutes bilateral renal ischemia with or without alpha-MSH treatment**

| | **ARF (n=9)** | **ARF + MSH (n=8)** | **Sham (n=9)** |
|---|---|---|---|
| ***p-creat (µmol*/*L)*** | 194 ± 45*' | 68 ±15* | 32 ± 1 |
| ***Ccr (ml*/*min)*** | 0.4 ± 0.1*' | 0.8 ± 0.1* | 1.3 ± 0.06 |
| ***FENa(%)*** | 4.2±0.6*' | 1.5±0.6 | 1 ±0.1 |

| ***Urine output (µl*/*min*/*Kg)*** | | | |
|---|---|---|---|
| **Baseline period** | | | |
| Day-2 | 44±1 | 35±3 | 44±4 |
| Day-1 | 40±3 | 36±4 | 42±4 |

| **After operation** | | | |
|---|---|---|---|
| Day 1 | 94±13* | 67±9* | 42±3 |
| Day 2 | 104±12*' | 71 ±8* | 51 ±2 |
| ***U*/*P osm*** | 1.7±0.3*' | 2.7±0.3* | 4.3±0.3 |
| ***TcH₂O* (µ*l*/*min*/*Kg)*** | 51 ±16*' | 118 ± 18* | 163± 10 |

| | | | |
|---|---|---|---|
| p-creat, Ccr, FENa, U/P osm, and TcH₂O were measured at Day 2. **P* < 0.05 when ARF rats, which were either treated or no' treated with α-MSH, were compared with sham operated rats. '*P* < 0.05 when ARF rats which were not treated with α-MSH were compared with ARF rats treated with α-MSH. | | | |

**Table 6. Changes in the abundance of major Na transporters by α-MSH treatment of rats with bilateral ischemia-induced ARF**

| | **ARF (n=9)** | **ARF + MSH (n=8)** | **Sham (n=9)** |
|---|---|---|---|
| ***Na,K-ATPase*** | 6 ± 3%*' | 91 ± 3% | 100 ± 8% |
| ***NHE-3*** | 3 ± 0.3%*' | 39 ± 13%* | 100 ± 14% |
| ***NaPi-2*** | 4 ± 1%*' | 48 ± 3%* | 100 ± 8% |
| ***BSC-1*** | 2 ± 0.2%*' | 59 ± 8%* | 100 ± 7% |
| ***TSC*** | 7 ± 1%*' | 87 ± 10% | 100 ± 12% |

| | | | |
|---|---|---|---|
| For densitometry of immunoblots, sodium transporter labeling was calculated as a fraction of the mean value from sham operated controls. **P* < 0.05 when ARF rats, which were either treated or not treated with α-MSH, were compared with sham operated rats. '*P* < 0.05 when ARF rats not treated with α-MSH were compared with ARF rats treated with α-MSH. | | | |

## Claims

1. Use of α-MSH or an α-MSH equivalent including the sequence His-Phe-Arg and EPO for the preparation of a medicament for treatment or prevention of an ischemic condition in the tissue of an organ of a mammal.

2. Use according to claim 1 wherein the condition is selected from the group consisting of atheromatous disease with thrombosis, embolism from the heart or from blood vessel from any organ, vasospasm, aortic aneurysm or aneurisms in other organs, thoracal or abdominal or dissecting aortic aneurysm, hypotension due to heart disease, hypotension due to systemic disease including infection or allergic reactions and hypotension due to one or more toxic compound or poison(s) or drug(s).

3. Use according to claim 1 wherein the condition is caused by ischemia secondary to a condition or disease selected from the group consisting of diabetes mellitus, hyperlipidaemia, thromboangiitis obliterans (Buerger's disease), Takayasu's syndrome, arteritis temporalis, mucocutaneous lymph node syndrome (Kawasaki disease), cardiovascular syphilis, connective tissue disorders, phlegmasia coerulae dolens, blood vessel trauma and organ transplantation.

4. Use according to claim 1 wherein the condition is caused by surgery of one or more organs, transplantation of one or more organs, surgical insertion transplants, devices, grafts, prostheses or other biomedical compounds or devices.

5. Use according to claim 1 wherein the ischemia is due to septic chock or conditions associated with systemic hypotension.

6. Use according to any of claims 1-5, wherein α-MSH or an α-MSH equivalent including the sequence His-Phe-Arg and EPO are to be administered independently of each other.

7. Use according to any of claims 1-5, wherein α-MSH or an α-MSH equivalent including the sequence His-Phe-Arg and EPO are to be co-administered.

8. A pharmaceutical composition comprising a combination of α-MSH or an α-MSH equivalent including the sequence His-Phe-Arg and EPO together with a pharmaceutically acceptable carrier, wherein said EPO is in the form of a recombinantly produced protein.

9. A kit comprising α-MSH or an α-MSH equivalent including the sequence His-Phe-Arg and EPO for sequential or simultaneous use in the treatment or prevention of an ischemic condition in the tissue of an organ of a mammal, wherein said EPO is in the form of a recombinantly produced protein.

## Patentansprüche

1. Anwendung von α-MSH oder einem α-MSH-Äquivalent einschließlich die Sequenz His-Phe-Arg und EPO zur Herstellung eines Arzneimittels zur Behandlung oder Verhinderung eines ischämischen Zustands im Gewebe eines Organs eines Säugetiers.

2. Anwendung nach Anspruch 1, worin der Zustand ausgewählt ist aus der Gruppe bestehend aus Gefäßwand-Erkrankungen mit Thrombose, Embolie des Herzens oder der Blutgefäße irgendeines Organs, Vasospasmus, aortischem Aneurysmus oder Aneurysmen in anderen Organen, thoracalem oder abdominalem oder seziertem aortischem Aneurysmus, Hypotension aufgrund von Herzerkrankungen, Hypotension aufgrund systemischer Erkrankung, einschließlich Infektion oder allergische Reaktionen, und Hypotension aufgrund einer oder mehrerer toxischen/toxischer Verbindung(en) oder eines oder mehrerer Gifts/Gifte oder Medikaments/Medikamente.

3. Anwendung nach Anspruch 1, worin der Zustand, verursacht durch Ischämie, sekundär zu einem Zustand oder einer Erkrankung ist, der/die ausgewählt ist aus der Gruppe bestehend aus Diabetes Mellitus, Hyperlipidaemia, Thromboangiitis Obliterans (Buerger-Krankheit), Takayasu-Syndrom, Arteritis Temporalis, mukokutanem Lympfknotensyndrom (Kawasaki-Krankheit), kardiovasculärer Syphilis, Funktionsstörungen des Bindegewebes, Phlegmasia Coerulae Dolens, Blutgefäß-Trauma und Organtransplantation.

4. Anwendung nach Anspruch 1, worin der Zustand durch Operation von einem oder mehreren Organ(en), Transplantation von einem oder mehreren Organ(en), operative Insertion von Transplantaten, Geräte, Transplantate, Protesen oder andere biomedizinische Verbindungen oder Geräte verursacht ist.

5. Anwendung nach Anspruch 1, worin die Ischämie durch septischen Shock oder Zustände, die mit systemischer Hypotension verbunden sind, verursacht ist.

6. Anwendung nach irgendeinem der Ansprüche 1-5, worin α-MSH oder ein α-MSH-Äquivalent einschließlich die Sequenz His-Phe-Arg und EPO unabhängig voneinander verabreicht werden muss.

7. Anwendung nach irgendeinem der Ansprüche 1-5, worin α-MSH oder ein α-MSH-Äquivalent einschließlich die Sequenz His-Phe-Arg und EPO mitverabreicht werden muss.

8. Pharmazeutische Zusammensetzung, umfassend eine Kombination von α-MSH oder einem α-MSH-Äquivalent, einschließlich die Sequenz His-Phe-Arg und EPO, zusammen mit einem pharmazeutisch verträglichen Träger, worin EPO in der Form eines rekombinant hergestellten Proteins ist.

9. Kit, umfassend α-MSH oder ein α-MSH-Äquivalent einschließlich die Sequenz His-Phe-Arg und EPO zur sequenziellen oder gleichzeitigen Anwendung zur Behandlung oder Verhinderung eines ischämischen Zustands im Gewebe eines Organs eines Säugetiers, worin EPO in der Form eines rekombinant hergestellten Proteins ist.

## Revendications

1. Usage d'α-MSH ou d'un équivalent α-MSH incluant la séquence His-Phe-Arg et l'EPO pour la préparation d'un médicament pour le traitement ou la prévention d'un état ischémique dans les tissus d'un organe d'un mammifère.

2. Usage selon la revendication 1 où l'état est choisi dans le groupe consistué par la maladie athéromateuse avec thrombose, l'embolisme du coeur ou du vaisseau sanguin d'un organe quelconque, le vasospasme, l'anévrisme aortique ou les anévrismes dans d'autres organes, l'anévrisme thoracal ou abdominal ou aortique disséquant, l'hypotension due à une maladie du coeur, l'hypotension due à une maladie systémique y compris les infections et les réactions allergiques et l'hypotension due à un ou plusieurs composés toxiques ou poison(s) ou drogue(s).

3. Usage selon la revendication 1 où l'état est causé par une ischémie secondaire à un état ou une maladie choisis dans le groupe consistué par le diabète mellitus, l'hyperlipidémie, la thromboangéite oblitérante (maladie de Buerger), le syndrome de Takayasu, l'artérite temporale, le syndrome lympho-cutanéo-muqueux (maladie de Kawasaki), la syphilis cardiovasculaire, les dysfonctionnements du tissu connectif, la phlegmatia coerula dolens, les traumatismes des vaisseaux sanguins et la transplantation d'organe.

4. Usage selon la revendication 1 où l'état est causé par l'opération chirurgicale d'un ou plusieurs organes, la transplantation d'un ou de plusieurs organes, l'insertion chirurgicale de transplants, d'appareils, de greffes, de prothèses ou autre composés ou appareils biomédicaux.

5. Usage selon la revendication 1 où l'ischémie est due à un choc septique ou aux conditions associées à l'hypotension systémique.

6. Usage selon une quelconque des revendications 1-5, où l'α-MSH ou un équivalent d'α-MSH incluant la séquence His-Phe-Arg et l'EPO doivent être administrés indépendamment l'un de l'autre.

7. Usage selon une quelconque des revendications 1-5, où l'α-MSH ou un équivalent d'α-MSH incluant la séquence His-Phe-Arg et l'EPO doivent être co-administrés.

8. Composition pharmaceutique comprenant une combinaison d'α-MSH ou d'un équivalent d'α-MSH incluant la séquence His-Phe-Arg et l'EPO avec un porteur acceptable pharmaceutiquement, où ladite EPO est sous la forme d'une protéine produite par un organisme recombinant.

9. Kit comprenant l'α-MSH ou un équivalent d'α-MSH incluant la séquence His-Phe-Arg et l'EPO pour usage séquentiel ou simultané dans le traitement ou la prévention d'un état ischémique dans les tissus d'un organe d'un mammifère, où ladite EPO est sous la forme d'une protéine produite par un organisme recombinant.
